# EUROPEAN PATENT APPLICATION

(11) **EP 3 926 034 A1**
(43) Date of publication of application: **22.12.2021**
(21) Application number: 20180088.5
(22) Date of filing: 15.06.2020
(51) Int. Cl.: C12M 1/36, C12M 3/00

(54) **SYSTEM FOR AUTOMATICALLY OPERATING A SMART TANK**

(71) Applicant: ESTR Biosystems GmbH, 37083 Göttingen (DE)
(72) Inventor: Schlack, Stefan, 37083 Göttingen (DE)
(74) Representative: Berger, Axel Bernhard

(57) **Abstract**

The present invention relates to smart tank for a bio-pharma process line, a smart tank assembly, a method for assembling a smart tank and a system comprising multiple smart tanks. The smart tank comprises a top plate element, at least one sidewall element, and a bottom plate element, wherein the top plate element, the at least one sidewall element and the bottom plate element are arranged to form a reservoir for receiving at least one biochemical medium. The smart tank comprises further at least one channel, for guiding the at least one biochemical medium and/or an operating medium.

## Description

### Field of the Invention

The present invention relates to a system for automatically operating a smart tank in a bio-pharma process line, a clean room bag, for being used in said system, a handling manipulator for automatically operating a smart tank in a bio-pharma process line and a method for automatically operating a smart tank in a bio-pharma process line. The system may be adapted for manufacturing and/or developing e.g. bio-pharmaceutical goods and/or for developing and/or testing the commercial manufacturing process of the same.

### Background Art

Bio-pharmaceutical goods, such as drugs, e.g. for cancer therapy, genetic therapy and/or cell therapy, are nowadays manufactured in so called bio-pharma process lines.

Known bio-pharma process lines are e.g. based stainless-steel tanks that are interconnected by pipes and/or the like. Those known bio-pharma process lines are difficult to maintain and to clean. Thus, so called single-use bio-pharma process lines were developed that are replaced after usage. Here, single-use containers, such as bags, serve as reservoirs for educts and/or products of the bio-pharma process line. Said bags are typically supported in rigid containers, such as stain-less steel containers, and are interconnected by hoses. For setting up a conventional single-use bio-pharma process line a complex manual assembly is required, leading to increased costs. For example, multiple bags, filters, sensors, valves, etc., have to be connected via various hoses. The complex assembly bears a significant risk for mal-function of the bio-pharma process line, due to improper or incorrect assembling.

Further, the single-use bio-pharma process lines a prone to damages as the (hose-based) connections and/or bags of the single use containers tend to leakages over the operational time. In case of leakage or mal-function, the educts and/or products of the entire bio-pharma process line can be damaged or even destroyed. Accordingly, there is a significant economic risk for the operator of the bio-pharma process line.

For operating a biopharma process line, bags or single use containers are typically provided with actuators, such as pump or pump heads that have to be controlled separately. Likewise, stirring means and/or valves may be provided with electric motors that need to be controlled separately. This requires a complex control scheme that cannot be easily adapted or extended.

Due to the complex built of the single-use bio-pharma process lines, automatization of assembling and operating the bio-pharma process lines is very difficult and expensive. Thus, in particular smaller bio-pharma process lines are oftentimes manually operated. This bears the risk of operation failures.

In view of the above, it is an object of the present invention to facilitate control and operation of a bio-pharma process line and to overcome the above-mentioned drawbacks. Further, costs and time required for developing and manufacturing bio-pharmaceutical goods shall be reduced, thereby allowing a faster time-to-market.

### Summary of the Invention

The object is achieved by a system for automatically operating a smart tank in a bio-pharma process line, a clean room bag, for being used in said system, a handling manipulator for automatically operating a smart tank in a bio-pharma process line and a method for automatically operating a smart tank in a bio-pharma process line.

Particularly, the object is achieved by a system for automatically operating a smart tank in a bio-pharma process line. The system comprises at least one smart tank, wherein the smart tank comprises an access plate element that forms a wall portion of a reservoir of the smart tank. Further wall portions of the smart tank may be formed by further rigid elements and or flexible elements, such as bags, foils, foil portions or the like. In case of flexible elements, the smart tank may be held in a support structure.

The reservoir of the smart tank is adapted to receive at least one biochemical medium, wherein the access plate element is configured to provide access to the smart tank.

The access plate element may allow to transfer biochemical medium into the smart tank and/or to remove by a biochemical medium from the smart tank. For example, biochemical medium may be removed for sampling. Likewise, operating medium can be transferred to the smart tank and/or can be removed from the smart tank via the access plate element. Sterilized pressurized air, an inert gas, or the like may serve as operating medium. Further, the access plate element may provide access to an actuating means of the smart tank that allow for example opening/closing a valve of the smart tank and/or to a driving a driving means that drives an operating means, such as a pump head or a stirring means. Further, the access plate element may provide a data interface that allows reading sensor data provided by a sensor or a sensor module of the respective smart tank.

The smart tank and in particular the reservoir may serve for storing and/or transporting a biochemical medium. Further, the reservoir may serve as a bioreactor. Additionally, or alternatively, the reservoir may include a filter such that the smart tank can be used for filtering.

The system comprises further at least one handling manipulator, wherein the handling manipulator is arranged movably with respect to the at least one smart tank so as to access and to control at least one smart via the access plate element. The smart tank may be automatically controlled by the at least one handling manipulator, by e.g. adding/removing operating and/or biochemical medium, by opening/closing valve(s) provided in the smart tank, and/or by driving an actuating means. The handling manipulator may carry out further tasks to control and operate the smart tank. Particularly, the handling manipulator may be configured to couple with the access plate of the smart tank to control and/or operate the smart tank.

The at least one manipulator may be arranged movably in all directions. Preferably, the at least one manipulator is provided on a guide rail(s) that allows movement of the manipulator in a horizontal and/or vertical plane. The manipulator may further be arranged movable in a direction perpendicular to a plane defined by the guide rails. For example, the at least one manipulator may be a portal robot that comprises a first guide rail that allows moving the manipulator in a x-direction and a second guide rail that allows moving the manipulator in a y-direction, wherein the x-direction is perpendicular to the y-direction. Additionally, or alternatively, the manipulator may comprise a third guide rail that allows moving the manipulator in a z-direction, wherein the z-direction is perpendicular to the x-direction and the y-direction.

In case the manipulator is moveable, several smart tanks can be controlled and operated using the same handling manipulator. Those smart tanks may be connected to form a smart tank system, e.g. of a bio-pharma process line. Thus, by controlling the smart tanks, the smart tank system and/or the bio-pharma process line can be controlled and operated using the handling manipulator. Particularly, the handling manipulator may be moved towards the smart tanks in a predefined order to carry out predefined control or operating tasks. Further, the handling manipulator maybe connected to a control unit that receives sensor signals from the respective smart tanks. The control unit may, based on the received sensor signals, generate manipulator commands that are sent to the at least one handling manipulator to operate and control the smart tank(s) according to a control scheme. For example, the pH value and/or a temperature of a smart tank can be measured. In case the pH value exceeds a predefined threshold, acid can be added by the handling manipulator (e.g. by injecting acid or by opening an acid supply valve) until the pH value meets a predefined condition. Further, in case a temperature drops below a predefined threshold, the handling manipulator may open a valve to allow more heating fluid to flow through a heating channel of the smart tank. Thereby, the smart tank and/or the smart tank system can be operated and controlled.

The system may comprise multiple handling manipulators (e.g. at least two, at least three, at least five or at least ten). Providing multiple handling manipulators allows simultaneous control/operation of multiple smart tanks. The multiple smart tanks may be controlled jointly, to carry out common operating tasks. For example, a pH value may be controlled while at the same time temperature is adjusted.

The multiple handling manipulators may be provided on separate guide rails or they may share at least one common guide rail. For example, the x-direction guide rail may be a shared guide rail, wherein each manipulator comprises a separate y-direction guide rail.

The at least one smart tank may be modular smart tank that is assembled from a top plate element, at least one sidewall element, and a bottom plate element. The top plate element, the at least one sidewall element and the bottom plate element are arranged to form at least one reservoir of the smart tank, wherein at least one of the top plate element, the at least one sidewall element and the bottom plate element is an access plate element. Optionally, the smart tank comprises at least one channel, for guiding the at least one biochemical medium and/or an operating medium, wherein the at least one channel extends within at least one of the top plate element, the at least one sidewall element and/or the bottom plate element.

Generally, all channels and/or reservoirs of the smart tank may be configured to be self-emptying. I.e. medium that has entered the channel and/or reservoir can flow out of the respective channel and/or reservoir by gravitation.

The at least one smart tank may comprise at least one of the following components: a port, a filter, a valve, a sensor and/or an operating means, and wherein at least one of these components is accessible with the handling manipulator via the access plate element.

Further, the assembled smart tank may comprise one reservoir or multiple (at least two) reservoirs, formed by the top plate element, at least one sidewall element, and a bottom plate element. Each of the top plate element, the at least one sidewall element, and the bottom plate element may serve as access plate element, i.e. the handling manipulator may access the smart tank via either one (or multiple) of the smart tank elements. In case of multiple reservoirs, the reservoirs maybe arranged serial or parallel to each other. Medium flowing through serial reservoirs flows through the single reservoirs one after another. Medium flowing through parallel reservoirs flows through the single reservoirs in a parallel fashion. Combinations of serial and parallel arrangement of the reservoirs is also possible. The flow of the medium through the one or multiple reservoirs may be controlled by the handling manipulator and in particular by opening/closing corresponding valves respectively.

The smart tank is adapted for being used in a bio-pharma process line, for manufacturing and/or developing bio-pharmaceutical goods and/or for developing and/or testing the commercial manufacturing process of these goods. Further, the smart tank may be used in other manufacturing lines and may be adapted to be operated automatically and/or manually. The bio-pharma process line and/or manufacturing line may be controlled by operating the smart tank(s) using the handling manipulator.

The biochemical medium and/or the operating medium may be any educt or product of the process line, such as sample containing medium, cell containing medium, drug containing medium, buffer medium, acids, bases, and/or the like. The medium may comprise at least one of the following: small molecule API, antibody, drug conjugates, RNA or fragments thereof, rec proteins, viral vaccines, bacterial/microbial processes, virus like particles, viral vectors, ADC, DNA, and/or the like. Further, the operating medium may comprise heating or cooling fluids, pressurized air or gases, and/or the like. For example, in case pressurized air is used as operating medium, the operating medium may serve for driving a biochemical medium through the channel and/or into (or out of) the reservoir of the smart tank.

The biochemical medium and/or the operating medium may be provided in liquid and/or gaseous form as well as in form of solutions and/or emulsions and/or suspensions. The mediums may be provided directly by the at least one handling manipulator, and/or may be received from further smart tanks or reservoirs that are connected to the respective smart tank to be operated. Medium flow from and to a smart tank can be controlled by opening/closing valves, using the handling manipulator.

Particularly, the operating medium, such as pressurized air, may be used to transfer a biochemical medium to and/or out of the smart tank. Therefore, the smart tank is operable via the operating medium. For transferring a biochemical medium out of the smart tank, a positive pressure can be applied (e.g. by the handling manipulator) to the first smart tank, by applying operating medium to the smart tank. Thus, biochemical medium is urged out of the smart tank, e.g. towards a second smart tank and/or a filter. For transferring biochemical medium into the smart tank, a negative pressure can be established, particularly by the handling manipulator, e.g. by removing medium from the smart tank. In case pressurized air is used as operating medium, preferably sterile pressurized air is used. Sterile pressurized air can be obtained by guiding pressurized air through a respective sterile filter prior to entering the smart tank. An operating medium, such as pressurized air can be supplied to or removed from the smart tank by a respective gas inlet and/or outlet port using the handling manipulator. The application and/or removal of medium from the smart tank may for example be performed pressure controlled, volume controlled, and/or mass controlled.

For pressure control a pressure sensor is provided that allows to measure the pressure within the reservoir of the smart tank. Medium can be supplied to the smart tank until a desired pressure is achieved. Likewise, medium can be removed from the smart tank until a desired lower pressure is achieved. For volume control a volume sensor, such as a capacitive volume sensor, maybe provided that allows monitoring the fill level (volume) of the smart tank. For mass control, the smart tank may be provided on a balance, such that the weight of the smart tank can be measured and controlled. Further, a mass flow controller may be provided that allows to measure and/or control a flow of gas.

The terms top plate element, side wall element and bottom plate element do not specify the orientation of the smart tank, when being assembled and in use. Rather, the assembled smart tank may be used in any orientation. Typically, the bottom plate element serves as a base and can optionally be provided on a stand for adjusting the height of the smart tank, when being in use/operation. For example, the orientation of the smart tank can be a standing orientation (i.e. the top plate element is on top), a lying orientation (i.e. a side wall element is on top), or an upside-down orientation (bottom plate element is on top).

Providing a top plate element, at least one side wall element and bottom plate element that are arranged to form a reservoir for receiving at least one biochemical medium facilitates cleaning and maintaining the smart tank after being used. Thus, the elements or at least some of the elements can be reused and/or recycled separately. Further, the smart tank - respectively the elements forming the reservoir - can be easily cleaned and sterilized prior to use. Optionally, the at least one side wall element may be integrally formed with the top plate element or the bottom plate element. Thus, assembling the smart tank is facilitated.

The at least one channel of the smart tank is configured for guiding at least one biochemical medium and/or an operating medium. Optionally, the smart tank may comprise multiple channels, wherein each of said channels may be adapted to guide a different biochemical medium and/or an operating medium, if the smart tank is in use. The at least one channel extends within at least one of the top plate element, the at least one sidewall element and the bottom plate element. Different channels may be provided in different or same elements, so that each of the top plate element, the at least one sidewall element and/or the bottom plate element may comprise at least one (or multiple) channels.

A channel is any inner lumen that extends in the top plate element, the at least one sidewall element and/or the bottom plate element and that is adapted to guide a flow of a biochemical medium and/or an operating medium. Particularly, the channel may be integrally formed with the respective element(s). The channel may be in communication with the reservoir of the smart tank and/or may bypass the reservoir of the smart tank. The channel may have a diameter in the range of 0.1 to 3 inch (0.25 to 7.6 cm), preferably in the range of 0.2 to 2 inch (0.5 to 5.1 cm), more preferably in the range of 0.3 to 1.5 inch (0.75 to 3.8 cm), even more preferably in the range of 0.5 to 1 inch (1.2 to 2.5 cm) and most preferably about 0.75 inch (1.9 cm). Further, different channels may have different diameters and/or the diameter of the channel may vary. Thus, e.g. nozzles maybe provided.

Particularly, a channel may extend, at least partially, in the top plate element, the at least one sidewall element and/or the bottom plate element, wherein the length of the channel is longer than the thickness of the respective top plate element, sidewall element and/or the bottom plate element. Accordingly, the medium may be guided by the channel in a direction that is different from a surface normal of a main surface of the respective top plate element, sidewall element and/or the bottom plate element.

Optionally, the medium transfer to and/or from the reservoir may be achieved exclusively by the at least one channel or multiple channels that extends in the top plate element, the at least one sidewall element and/or the bottom plate element. By transferring the medium using the channel(s) of the smart tank elements, the number of different contact materials can be reduced to a minimum and lot to lot consistency of the product line can be increased.

The at least one channel may extend in the at least one sidewall element and at least one of the top plate element and the bottom plate element. Thus, a biochemical medium maybe removed from a lower portion of the smart tank (e.g. via a first end of the channel, being provided in the bottom plate element or in a sidewall element in proximity to the bottom plate element) and guided to an upper portion of the smart tank (e.g. via a second end of the channel, being provided in the top plate element or in a sidewall element in proximity to the top plate element) e.g. for being transferred into a further smart tank. Accordingly, a biochemical medium may be removed from an upper portion of the smart tank (e.g. via a first end of the channel, being provided in the top plate element or in a sidewall element in proximity to the top plate element) and guided to a lower portion of the smart tank (e.g. via a second end of the channel, being provided in the bottom plate element or in a sidewall element in proximity to the bottom plate element) e.g. for being transferred into a further smart tank. Further, an operating medium, such as a heating or cooling medium may be guided within the at least one sidewall element and at least one of the top plate element and the bottom plate element, so as to provide a proper cooling/heating of the smart tank. The heating or cooling medium may be guided within the top plate element, the at least one sidewall element and/or the bottom plate element without being in contact to the reservoir. Thus, the heating or cooling medium and the bio-chemical medium are separated from each other. The respective heating/cooling channels of the smart tank maybe provided with flow control valves that allow to control the flow of the heating/cooling medium, preferably via the handling manipulator. Thus, the temperature of the smart tank can be controlled.

Generally, the smart tank may serve as reservoir for any educt or product of the process line, such as a biochemical medium and/or an operating medium. Thus, the received medium may be stored and/or transported. Further, the smart tank may be configured to blend different mediums received in the reservoir of the smart tank. Further, the smart tank may serve as bioreactor and may support a biologically active environment. For example, a chemical process which involves organisms or biochemically active substances derived from such organisms can be carried out in said smart tank. Further, the smart tank may be designed to grow cells or tissues in the context of a cell culture. The smart tank may be used as a batch bioreactor, a fed batch bioreactor, a concentrated fed batch bioreactor, or a continuous bioreactor and/or a perfusion bioreactor. Additionally, or optionally, the smart tank may serve as filtration unit for filtering the medium received in the reservoir of the smart tank. In this case, a filter may be provided in or on the top plate element, the at least one sidewall element and/or the bottom plate element. Further, the smart tank may serve for preparing a biochemical medium, such as a buffer medium and/or for carrying out a preparative chromatography. Further, the smart tank may comprise a cross flow cassette and/or a hollow fibre module, for filtration of virus, cell harvesting and/or ultra or dia-filtration.

The reservoir of the smart tank may have a volume of at least about 10 ml, of at least about 15 ml, of at least about 20 ml, of at least about 50 ml, of at least about 100 ml, of at least about 200 ml, of at least about 250 ml, of at least about 500ml, of at least about 1 l, of at least about 2 l, of at least about 5 l, of at least about 10 l, of at least about 20 l, of at least about 50 l, of at least about 100 l, of at least about 200 l, of at least about 500 l, of at least about 1000 l, of at least about 2000 l, of at least about 3000 l, of at least about 4000 l, or of at least about 5000 l. Thus, bio-pharma process lines can be scaled. For example, during development smart tanks with a small volume are used. Afterwards, during manufacturing, larger smart tanks are used. Further, smart tanks of different volumes can be combined in a smart tank system of a bio-pharma process line.

The at least one channel maybe chosen from a group of different channel-types, comprising the following channel types: an inlet channel, an outlet channel, a bypass channel, a heating or cooling channel, a sampling channel, or the like. An inlet channel serves for guiding a biochemical medium and/or an operating medium to the reservoir of the smart tank.

The inlet channel may comprise a sparger or may be coupled to a sparger.

An outlet channel serves for removing a biochemical medium and/or an operating medium from the reservoir of the smart tank. When being connected to a further (second) smart tank, the outlet channel of a first smart tank may be connected to an inlet cannel of the second smart tank, so as to transfer medium from the reservoir of the first smart tank to a reservoir of the second smart tank.

Further, a channel may be provided that serves for transferring a retentate from e.g. a filter or a membrane back into the smart tank. This channel may be a separate retentate channel or said channel may be integrally formed with an outlet channel. For transferring a retentate back into the smart tank, a filter or membrane can be flown through with an operating fluid, such as pressurized air, in a direction opposite to the operating direction. The operating direction is the direction in which the medium flows for filtering. During filtering, permeate goes through the membrane/filter, retentate is hold back by the membrane/filter.

A further channel may be provided that serves for transferring permeate and/or filtrate back into the reservoir and/or to another smart tank. For transferring a permeate and/or filtrate back into the reservoir and/or to another smart tank, a positive pressure may be applied on a permeate (filtrate) side of a filter. This may be achieved by guiding an operating medium, such as pressurized (sterile) air, to the permeate side. The operating medium urges the permeate/filtrate towards a permeate channel or a filtrate channel, respectively, that may then guide the permeate/ filtrate back into the reservoir of the smart tank and/or to another smart tank. The direction of flow can be controlled by opening/closing respective valves that can be associated with the respective channels.

A bypass-channel serves for guiding a biochemical medium and/or an operating medium, wherein the bypass-channel is not connected to the reservoir of the smart tank. Thus, a medium can bypass the smart tank without being in communication with the reservoir of the smart tank. For example, when three smart tanks are connected so that a first and a third smart tank sandwich a second smart tank, a fluid may be guided from the reservoir of the first smart tank to a reservoir of the third smart tank without passing a reservoir of the second smart tank. Optionally, the bypass-channel may be adapted to be fluidically separated from the reservoir of the smart tank. This can be achieved e.g. by a valve. Depending on the valve position (open/closed), the bypass-channel may be or may not be in communication with the reservoir of the smart tank. Thus, the flow of the medium can be controlled in that the medium bypasses the reservoir of the smart tank, or not.

For example, the bypass-channel allows to provide a biochemical medium, such as a buffer, that is used in various smart tanks of a bio-pharma process line in a large storage smart tank. Said biochemical medium can then be transferred from the storage smart tank to all smart tanks that require said medium (e.g. buffer). In case a smart tank does not require said medium, the medium can bypass the reservoir of said smart tank. Further, by providing a valve in the bypass channel, the supply of said medium can be controlled (e.g. amount and time), preferably by using the at least one handling manipulator. Further, the bypass-channel may serve for transferring a bio-chemical medium, such as a cell culture, from a first smart tank (e.g. a seed tank) in at least two subsequent smart tanks that may serve as further bioreactors. Thus, a bio-chemical medium can be guided from a single tank to multiple subsequent tanks.

Generally, the flow of medium through the smart tank and/or channels of the smart tank can be controlled by opening/closing respective valves and by applying positive and/or negative pressure to respective ports, channels and/or reservoirs of the smart tank, preferably by using the at least one handling manipulator.

A sampling channel serves for taking a sample of biochemical medium and/or of operating medium from the reservoir of the smart tank. Thus, educts, products and intermediate products or the process line can be removed and analyzed. A sample may be removed from the smart tank utilizing the at least one handling manipulator.

The smart tank may comprise multiple channels of different channel-types and/or the same channel type. Thus, the functionality of the smart tank can be adapted to the specific needs of the respective process line. In particular, the smart tank may be a multi-functional smart tank comprising multiple channels of different channel-types and/or the same channel type. At least one channel may be opened and closed, using e.g. a valve, a closure, or the like, preferably by the handling manipulator. Thus, depending on desired functionality a channel or multiple channels can be closed. These closed channels are not used (at least temporarily). At least one other or multiple other channels may be opened and therefore used (at least temporarily), thereby defining the functionality of the multi-functional smart tank.

The smart tank may comprise at least one port, wherein the at least one port may be associated with a respective channel. The port may be chosen from a group of port-types, comprising the following port-types: a fluid inlet port, a gas inlet port, a fluid outlet port, a gas outlet port, a cell blead port, a tank-interconnecting port, an element-interconnecting port, a medium supply port, a medium remove port and/or the like.

The (fluid or gas) inlet port serves for providing a biochemical medium and/or an operating medium to the reservoir of the smart tank. A (fluid or gas) outlet port serves for removing a biochemical medium and/or an operating medium from the reservoir of the smart tank. A medium supply port allows to supply medium (biochemical medium and/or an operating medium) to the smart tank and a medium remove port allows to remove medium (or parts thereof) from the smart tank. The medium remove port may also server for transferring a retentate from e.g. a filter or a membrane back into the smart tank. A tank-interconnecting port serves for coupling a channel of a first smart tank fluidically to a corresponding channel of a second smart tank, when the first smart tank is connected with the second smart tank, e.g. by using a connector means as will be described in greater detail below. A tank-interconnecting port may serve as medium supply port and/or medium remove port. A cell blead port serves for removing cells from the reservoir. The cells may either be discarded or may be transferred to a further smart tank or other device for further processing. The at least one port may be associated with a valve that can preferably be controlled by the handling manipulator. Thus, the flow of medium from and to the smart tank can be controlled by opening/closing the valve. Particularly, the valve maybe a flow control valve, that allows to adjust the flow gradually.

For example, a channel may be associated with a tank-interconnecting port and a fluid outlet port. Thus, said channel may serve for transferring a medium, such as a fluid, from the reservoir of the smart tank to a further smart tank. In case a valve is associated with the tank-interconnecting port, the flow of the transferred medium can be controlled. Alternatively, or additionally, the flow may be controlled by controlling the pressure in the associated smart tanks (positive and/or negative pressure).

Further, the port (e.g. a tank-interconnecting port or an element-interconnecting port) maybe adapted to engage with a corresponding port (e.g. a corresponding tank-interconnecting port or an element-interconnecting port), wherein the port and the corresponding port are formed to provide a positive locking. For example, the smart tank may comprise a port on a first side and a corresponding port on a second side, wherein the first side and the second side may be opposing outer surfaces of the smart tank. This allows to couple the smart tank with a further smart tank using the port and the corresponding port and to fluidically connect the respective channels when coupling the smart tanks. Thus, a medium can be transferred from a first smart tank to a second smart tank without the use of intermediate conduit elements, such as pipes or hoses. Thereby assembling a smart tank system of e.g. a bio-pharma process line is facilitated and less prone to assembly mistakes.

The ports may include a sealing member (e.g. flexible sealing member, such as a rubber seal, a silicon seal, a Teflon seal, or the like). Said sealing member may be a radial and/or an axial sealing. For example, the sealing member maybe provided on a shroud and/or within a recess of the port. The sealing member maybe provided as sealing gasket that allows to seal multiple ports of the smart tank. Further, the sealing member may be port specific and adapted to seal only a single port.

The smart tank may comprise at least one filter, wherein the least one filter may be chosen from a group of filter-types, comprising the following filter-types: a pre-filter, a sterile filter, a bacterial filter, a viral filter, a mycoplasma filter, an ultrafiltration filter, a diafiltration filter, a cell filter, a fluid filter, an air filter, a gas filter, or the like. The filter may be provided within the reservoir of the smart tank. Thus, the smart tank may serve as filtration unit for filtering the medium received in the reservoir of the smart tank.

Further, at least one port of the smart tank may be covered by a filter. Providing a filter-covered port allows to withhold parts of the medium in the smart tank and/or prevent other parts of a medium from entering the smart tank. In case an inlet port is covered with a filter, only filtrate can enter the smart tank. In case an outlet port is covered, only the filtrate is allowed to leave the smart tank. For example, a gas inlet port may be covered with a sterile filter. Thus, sterile pressurized air can be guided into the tank and thus, medium contained in the reservoir of the smart tank can be blown out of the reservoir, e.g. for being transferred to a further smart tank. Thus, the flow into and out of a smart tank can be controlled.

Further, the filter covering the at least one port may be heated and/or cooled. Thus, medium entering and/or leaving the smart tank can be tempered to a desired temperature. Further, heating the filter allows to provide a hot filtration and cooling the filter allows to provide a cold filtration. Heating a filter further serves to prevent undesired condensation.

The smart tank may comprise at least one valve. The at least one valve may be associated with the at least one channel and/or the at least one port. The at least one valve may be a flow control valve, a cutoff valve, a pressure relief valve or a non-return valve, or the like. Further, a smart tank may comprise multiple valves of the same and/or of different types. Particularly, a valve maybe provided at a junction of at least two channel portions. Thus, depending on the valve position, medium can be guided to different channels and/or tanks.

A flow control valve allows to control the amount of medium flowing through the channel per unit of time (e.g. flow in liters/second). The flow control valve may be configured to control the flow dynamically, to control a biochemical process in the smart tank. A cutoff valve allows to open or close the channel. A cut off valve may be provided in a bypass-channel. Thus, the bypass-channel may be fluidically separated from the reservoir of the smart tank (valve closed) of in communication with the reservoir of the smart tank (valve open). Further a cutoff valve allows to close channels that are not used and open channels that are used in the smart tank, depending of the functionality of the smart tank.

A non-return valve maybe provided in a channel to prevent medium that shall be removed from the smart tank to flow back into the smart tank. Thus, e.g. contamination of the smart tank can be prevented. Further, a non-return valve may prevent medium that has entered the smart tank from flowing back to its origin. Thus, a smart tank can be pressurized (e.g. by pressurized air) and medium contained in the reservoir of the smart tank can be guided in a desired direction, for example for filtration purposes. Using pressurized smart tanks allows to guide the medium without using pumps. Thus, contamination of the medium and particle generation (e.g. due to abrasion) can effectively be reduced or even prevented. Further, no (expensive) pumps, such as single use pumps or pump heads, are needed, thereby reducing the costs of the smart tank and/or the smart tank system for a bio-pharma process line. Still further, avoiding pumps facilitates cleaning, maintaining and/or sterilizing the smart tank.

The at least one valve can have any suitable configuration. For example, the at least one valve can be a ball valve, a butterfly valve, a diaphragm valve, a gate valve, a needle valve, a pinch valve, or the like.

The at least one valve may be configured to be actuated manually and/or automatically. For example, the valve can be configured to be actuated mechanically, pneumatically, hydraulically, magnetically, electrically, and/or the like. Further, the at least one valve may be configured to be actuated from the outside of the smart tank, by means of an actuating means. In particular, the valve may be a mechanical valve that is configured to be actuatable from the outside of the smart tank, by means of an actuating means. The actuating means may be an actuating rod, that connects a valve closure member that is in contact with the medium with the outside of the smart tank. The actuating rod maybe supported and sealed in at least one element (top plate element, sidewall element, bottom plate element) of the smart tank. For opening/closing the valve, the actuating means can be rotated or axially displaced, depending on the type of associated valve. The actuating means may be adapted to couple with an actuating mechanism, that maybe part of the handling manipulator that allows automated control of the actuating means and thus of the smart tank and/or a smart tank system.

Further, the actuating means may include a magnet (permanent or electrical) that allows to actuate a respective magnetic valve from the outside of the smart tank. Providing a magnetic actuating means facilitates the sealing of the valve, as the valve closure member and the actuating means can be separated from each other, e.g. by a continuous wall portion. Further, the magnetic actuating means may couple magnetically with the drive mechanism. Thus, the actuating means can be controlled from the outside of the smart tank. Particularly, when using mechanical and/or magnetically valves it is possible to avoid electric valve components being integrated in the smart tank. Thus, recycling of the smart tank is facilitated and improved.

The smart tank may further comprise at least one pumping means, wherein the pumping means may be separated from the reservoir and/or the at least one channel by a flexible membrane, so as to prevent direct contact between the at least one biochemical medium and the pumping means. The pumping means may be operable using the handling manipulator.

The smart tank may further comprise at least one stirring means, wherein the stirring means may be drivable from the outside of the smart tank. For example, the stirring means may be driven by a magnetic actuator. Using a magnetic actuator allows to drive the stirring means from the outside of the smart tank and facilitates the sealing, as the magnetic actuator can be separated from the actual stirring means, e.g. by a continuous wall portion. The magnetic actuator may be part of a handling manipulator and thus, the handling manipulator maybe able to control the stirring means (e.g. stirring velocity, stirring time, stirring duration, ...).

Further, the stirring means may comprise an actuating rod. Said actuating rod may be supported and sealed in at least one element (top plate element, sidewall element, bottom plate element) of the smart tank. The actuating rod can be engageable with a drive mechanism, such as an electric drive mechanism, provided on the outside of the smart tank. The drive mechanism may be part of the handling manipulator that allows automated control of the smart tank and/or a smart tank system.

The stirring means may comprise at least one stirring member, wherein the stirring member comprises multiple stirring blades. The stirring blades can be provided in different forms, such as in form of a Rushton stirring blade, a pitched blade, a gentle marine blade, or the like. Further, the stirring means may comprise multiple stirring members, being e.g. provided in different levels of the smart tank. Further, the stirring means may include an integrated sparger.

The stirring means may be operable in a pulsed mode. Thereby waves are created that allow to clean up a filter from a reservoir side. Further, for cleaning up a filter, a further stirring member may be provided that is adapted to rotate in close proximity to the filter. Depending on the type of filter, the reservoir side may be a permeate side or a retentate side of the filter.

The smart tank may further comprise at least one a cell-harvest-means. The cell-harvest means may include a filter and/or a filter cartridge that can be coupled or integrated in an element, particularly the bottom plate element of the smart tank. The cell-harvest-means may comprise medium remove port (also referenced as cell bleed port), for removing the medium or parts of the medium (such as cells) contained in the reservoir of the smart tank. The medium remove port may also be provided in the bottom plate element and/or the at least one side wall element. In particular, the medium remove port can be provided anywhere on a reservoir-side of a filter of the cell-harvest-means for removing retentate, such as cells, and/or on the opposite side, for removing filtrate (permeate). Further, the cell-harvest-means may include an (operating) medium supply port, that allows to rinse a filter of the cell-harvest-means.

The operating medium supply port may be provided on the permeate side of the filter. Thus, when positive pressure (gaseous or fluidic) is applied on the permeate side of the filter, e.g. via the (operating) medium supply port, retentate can be transferred back into the smart tank and/or out of the medium remove port. Further, for transferring retentate back into the smart tank and/or out of the medium remove port a negative pressure can be applied on the retentate side of the filter.

Additionally, applying positive pressure (gaseous or fluidic) on the permeate side of the filter or the filtrate side of the filter, e.g. via the (operating) medium supply port, permeate/filtrate can be urged into a permeate channel or a filtrate channel, respectively and thus, permeate/filtrate can be guided back into the reservoir and/or to a further smart tank. Preferably, positive pressure is applied by providing sterile pressurized air on the permeate side, or the filtrate side respectively
The smart tank may further comprise at least one cartridge for chromatography, so as to allow carrying out a preparative chromatography. Further, the smart tank may comprise at least one resin means, a membrane absorber, and/or the like. The smart tank may further comprise at least one cross-flow cassette, so as to allow carrying out crossflow filtration or tangential flow filtration within the smart tank.

The smart tank may further comprise at least one hollow-fibre means for cell harvesting, dia-filtration, mirco-filtration, ultra-filtration, and/or the like. The hollow-fibre means may be provided in a cartridge that can be integrated in the reservoir of the smart tank or that can be arranged outside of the reservoir of the smart tank. For example, the hollow-fibre means may be coupled to at least one of the elements of the smart tanks, using respective ports. Further, the smart tank may comprise multiple hollow-fibre means. Particularly, a smart tank may be used for increasing the cell concentration of a cell containing solution, e.g. by filtering the solvent. This allows to harvest cultivated cells.

The at least one sidewall element and/or the bottom plate element may be integrally formed with either one of the above-mentioned filter, cell-harvest-means, cartridge for chromatography, cross-flow-cassette, resin means, hollow-fibre means, and or any other fluid storing or guiding component.

The smart tank may be connectable to at least one sensor or a sensor module, comprising multiple sensors, wherein the at least one sensor and the sensors of the sensor module are chosen from the group of a pH sensor, a temperature sensor, a dissolved oxygen sensor, a biomass sensor, a foam sensor, a pressure sensor, a flow sensor, an O₂ sensor, an N₂ sensor, a CO₂ sensor, or the like. Further, the at least one sensor may be a spectroscopy means, such as RAMAN, NIR and/or UV spectroscopy means. Further, the sensor or sensor module may be connected to the smart tank.

For measuring pressure within a smart tank and/or a channel thereof, a channel may include a flexible membrane. This membrane can be associated with a pressure sensor that is adapted for the pressure inside the tank and/or the channel. Further, the pressure within the tank and/or the channel may be measured, using a hydrophobic filter, such as a vent filter, that is installed in the top plate element. This filter can be separated from the reservoir of the tank by means of a valve. For measuring the pressure, the valve can be opened.

Being able to measure the pressure in a smart tank and/or a channel thereof, allows for leakage testing of the smart tank and/or for non-destructive integrity testing of filter(s) provided in the smart tank. Prior to leakage testing of the smart tank, the smart tank is pressurized, e.g. by applying pressurized air, preferably by a handling manipulator. Subsequently pressure drop can be measured and compared to a respective predefined pressure value. Thus, leakage can be detected. In case the measured pressure drop exceeds the predefined pressure values, the test is not passed.

For testing integrity of a filter, all input- and output channels of the smart tank should be closed, e.g. by using respective valves. Closing the valves is preferably carried out by the handling manipulator. Further, the filter should be wetted prior to testing. Wetting the filter can be achieved by opening a wetting channel that is associated with the filter to be tested. The wetting channel may guide a buffer solution to the filter, that wets the filter. Subsequently, the tank, particularly a permeate side (filtrate side) and/or a retentate side (upstream side) of the filter, may be pressurized and pressure drop over time can be measured. For pressurizing positive and/or negative pressure may be applied so that there is a differential pressure applied on the filter. The measured pressure drop can be compared to a predefined threshold to decide, whether the integrity test is passed or not. This integrity test can be performed for every filter of the tank. Integrity can then be tested e.g. using a pressure hold test, a pressure drop test and/or a forward flow test.

For testing integrity, the following steps can be carried out:
a) The filter(s) that shall be tested is wetted with a wetting fluid, e.g. by applying a buffer solution. The buffer solution may be applied through a buffer containing cartridge or smart tank, preferably by using the handling manipulator, i.e. automatically. For wetting the filter(s) a channel that fluidically connects the buffer containing cartridge or smart tank with the smart tank that contains the filter to be tested is opened, e.g. by opening a respective valve and/or by connecting the buffer containing cartridge or smart tank to the smart tank that contains the filter to be tested.
   Operating the valve(s) and connecting the buffer containing cartridge may be carried out by the handling manipulator.
   Further it may be monitored, whether wetting is completed. Completion of the wetting step can be detected e.g. by detecting permeate and/or filtrate that has travelled through the filter to be tested. This can be done by weighing, by providing a fluid sensor, such as a capacitive sensor, and/or the like. Additionally, or alternatively, the wetting fluid and respectively pressure, maybe provided for a predetermined period of time that guarantees a proper wetting of the filter(s). Excess wetting fluid can be removed via a waste channel and e.g. guided to waste tank. Further, if needed back pressure can be applied via the valves for improving the wetting of the filter.
b) After wetting is completed, e.g. the permeate side (filtrate side) and/or a retentate side (upstream side) of the filter is pressurized, preferably by applying sterile pressurized air. Prior to pressurizing, all valves that would allow pressurized air to leave the side of the filter that is pressurized should be closed, preferably by a respective handling manipulator.
   For testing membrane filters, typically the upstream side of the filter is pressurized. On the filtrate side of the filter a channel maybe opened, e.g. a waste channel, preferably by actuating a respective valve. This allows pressurized air that has travelled though the filter to be removed from the filtrate side.
   For testing a cross flow cassette and/or a hollow fibre module pressure may be applied on the retentate side, preferably via a retentate outlet channel of the smart tank. The feed channel(s) of the cross flow cassette and/or a hollow fibre module maybe closed for integrity testing and the permeate channel(s) of the cross flow cassette and/or a hollow fibre module may be opened for integrity testing. On the permeate side of the filter a channel may be opened, e.g. one of the permeate channels, and connected to a waste channel, preferably by actuating a respective valve. This allows pressurized air that has travelled though the filter to be removed from the permeate side.
c) After or during pressurizing, the pressure drop over time can be measured and compared to a predetermined threshold. Preferably, measuring is started after a predefined stabilization time. In case the pressure drop exceeds the threshold, the test is not passed.

After testing, the pressurized air may be removed from the smart tank via an outlet port.

Integrity testing is extremely difficult in conventional bag-based process lines, as the bags and/or hose connections expand due to the applied pressure. And therefore, the test result is distorted. Thus, in conventional bag-based process lines expensive helium leakage tests are required. Further, helium leakage testing is complex, and typically cannot be carried for interconnected bags of a conventional bag-based process lines, or even an entire bio-pharma process line. Particularly, interconnections of bags cannot be tested. Thus, being able to use integrity testing in a readily assembled process line and/or a readily assembled smart tank significantly reduces the risk of leakage. Further, costs for operating a process line.

The sensor and/or sensor module of the smart tank may comprise a wired or wireless data transfer unit for transferring achieved sensor data to a control unit. The control unit may then generate commands for operating/controlling the at least one handling manipulator, thereby operating/controlling the at least one smart tank. Further, the sensor and/or sensor module may comprise a rechargeable battery and/or a power interface for powering the sensor and/or the sensor module. The power interface may be wired or wireless, such as an inductive or capacitive power interface. Further, the sensor and/or sensor module may be configured for multi-use. Particularly, the sensor and/or sensor module may be sterilizable.

Further, a volume of the first smart tank may be smaller than a volume of the second smart tank and the first smart tank may be adapted to be installed on top of the second smart tank. The first and second smart tanks may be fluidically connected, wherein the fluid connection (channel) may be controlled by a valve that is preferably operable by at least one handling manipulator. The first smart tank may serve to provide an operating medium and/or a biochemical medium to the second smart tank. As the first smart tank is installed on top of the second smart tank, it is possible to transfer fluid from the first smart tank to the second smart tank by using gravitation. Thus, the use of a pump can be avoided.

The at least one (smaller) smart tank may be provided in a non-sterile (non-clean room) environment wherein the assembled smart tank is placed in a sterile or clean room environment. A barrier may separate the non-sterile (non-clean room) environment from the sterile or clean room environment, wherein the barrier (such as a foil portion) is sandwiched between the at least one (smaller) smart tank and the assembled smart tank. According to this alternative, the at least one (smaller) smart tank may be positioned on top of an assembled smart tank via the handling manipulator. Both smart tanks may be fluidically connected, via a channel or port. The channel or port may comprise a sterile filter, allowing to transfer medium from the smaller smart tank to the larger smart tank without contaminating the larger smart tank. The fluid connection may be controlled by a valve. The sterile filter may be arranged at least partially in a top plate element of the larger smart tank.

Further, medium can be transferred from a first smart tank to a second smart tank by pressurizing at least one of the smart tanks (positive or negative). In case medium shall be transferred from the first to the second smart tank, a positive pressure can be applied to the first smart tank and/or a negative pressure can be applied to the second smart tank. Thus, medium is urged from the first smart tank towards a second smart tank. In case a filter is provided between the first smart tank and the second smart tank (e.g. in an interconnection channel, and/or in a top plate element of the second smart tank, in case the first smart tank is installed on top of the second smart tank), filtration can be controlled by controlling the pressure level in the first and/or second smart tank. A positive pressure can be established by providing (operating) medium, such as pressurized air, to the smart tank, preferably by the at least one handling manipulator. A negative pressure can be established by removing (operating) medium from the respective smart tank, preferably by the at least one handling manipulator. In case pressurized air is used as operating medium, preferably sterile pressurized air is used. Sterile pressurized air can be obtained by guiding pressurized air through a respective sterile filter prior to entering the smart tank.

The system may further comprise at least one clean room bag that comprises at least one foil portion. The at least one clean room bag is configured to receive at least one smart tank and to provide a clean room or sterile environment for the at least one smart tank when the at least one smart tank is received in the clean room bag. Further, the at least one smart tank may be accessible with the handling manipulator via the access plate element, from the outside of the clean room bag.

A clean room bag can be installed in almost every environment, e.g. in a conventional factory hall. Thus, expensive and time-consuming planning and constructing of pharma-process line plants can be avoided. Thus, the bio-pharma process line can be set up very quickly. Further, as the clean room bag as such provides at least clean room conditions, no expensive conventional clean rooms have to be installed. Further, in case of a single-use clean room bag, expensive and time-consuming cleaning and testing can be avoided.

Preferably, the clean room bag is a flexible bag that requires little storing space, when not in use. The clean room bag can be pre-sterilized and inflated/unfolded when in use. Thus, a sterile or at least clean room environment can be provided.

The clean room bag provides at least clean room conditions. The clean room bag may provide at least ISO 8 (ISO 14644-1 and ISO 14698) conditions. Depending on the required environment, clean room conditions up to ISO 1 are also possible. More preferably, the clean room bag provides a sterile environment for operating the smart tank(s). The clean room bag may be sterilizable, e.g. by ETO, gamma sterilization or autoclaving.

The system may further comprise at least one adaptor plate element, wherein the adaptor plate element is associated with at least one smart tank. Particularly the adaptor plate element may be associated with a single smart tank or with multiple smart tanks. The adaptor plate element can be installed on the at least one smart tank, so that one of the at least one foil portions of the clean room bag is sandwiched between the access plate element of the smart tank and the adaptor plate element. Further, the adaptor plate element is configured to define at least one access point to provide access to the smart tank from the outside of the clean room bag, via the access plate element.

Accordingly, the smart tank can be provided in a clean room or sterile environment and at the same time can be accessed from the outside of the clean room bag. Thus, the handling manipulator and other control or operating devices can be provided outside of the clean room bag. Thus, the size of the clean room bag can be minimized.

The adaptor plate element may be configured to cover a filter and/or a port of the smart tank at least partially. Thus, medium (bio-chemical and/or operating medium) can be transferred to and/or removed from the smart tank from the outside of the clean room bag. A foil portion of the clean room bag that is sandwiched between the access plate element of the smart tank and the adaptor plate element may be provided removably and/or pierceable in an area of the filter and/or port of the smart tank. Thus, access to the smart tank can be provided from the outside of the clean room bag.

For accessing a smart tank from the outside of a clean room bag, the handling manipulator may carry out the following steps:
Step 1: Install an adaptor plate element on the at least one smart tank, so that one of the at least one foil portions of the clean room bag is sandwiched between the access plate element of the smart tank and the adaptor plate element. Optionally, remove a cover element, such as a cap, from a through hole (access point) of the adaptor plate element.
Step 2: Perforate the at least one foil portion of the clean room bag with a perforating means, such as a knife, needle, laser and/or the like. The perforation may be carried out in a way, that a foil portion is separated from the clean room bag.
Step 3: Optionally, gripping the separated foil portion using a gripping means, such as a vacuum gripper of the handling manipulator.
Step 4: Optionally,Welding the remaining foil portion with the adaptor plate element and/or the top plate element, preferably by using the handling manipulator.
Step 5: Optionally, placing a connector means, such as a quick connector means on the adaptor plate in an area of the removed/perforated foil portion.The adaptor plate element may be configured to support an actuating means of a valve and/or an operating means (such as a stirring means) of the smart tank, and wherein the foil portion may be provided removably and/or pierceable in an area of the actuating means of a valve and/or an operating means of the smart tank to provide access to the smart tank. Access maybe provided using the handling manipulator, as described above. Thus, a valve or an operating means, such as a stirring means, can be operated from the outside of the clean room bag.

Further, the clean room bag may be adapted to sealingly couple to an assembly room that servers for assembling at least one smart tank, wherein the clean room bag may be further configured to receive the at least one assembled smart tank from the assembly room. The assembly room may also provide a clean room and/or sterile environment for assembling the smart tank(s). Thus, the risk for contamination of the smart tank(s) during assembly is reduced.

The clean room bag may be a sterile bag, that is optionally a single-use bag. The clean room bag may be initially sterilized or maybe sterilizable at the site of the bio-pharmaceutical goods manufacturer. By providing a single-use bag, the risk for contamination of the smart tank(s) is further reduced. Still further, cleaning the clean room bag is not required after use.

The clean room bag may be held by a support structure. The support structure may be a rigid support structure, such as a frame. Alternatively, the support structure may be inflatable. It is also possible, that the clean room bag as such can be inflated. Thus, a mobile and self-standing clean room can be provided.

The clean room bag may comprise a multi-ply outer shell, wherein a first ply of the shell is the outermost ply, and wherein a second ply of the shell is the innermost ply, The space surrounded by the second ply of the shell may be pressurized with a pressure being lower than a pressure provided between the first and the second ply of the shell. A colored gas may be provided between the first and the second ply of the shell. Thus, when the second ply of the shell would leak, colored gas would enter the space surrounded by the second ply. This can be detected and hints at entry of contaminants in the sterile and/or clean room environment.

The clean room bag may include an sealable opening that is configured for receiving assembled smart tanks, wherein the sealable opening of the clean room bag may be configured to open jointly with a corresponding sealable opening of an assembly room, when the clean room bag is sealingly coupled to the assembly room. Further, the clean room bag may include multiple sealable openings. Thus, transferring assembled smart tanks from the assembly room to the clean room bag is facilitated.

The sealable opening of the clean room bag may be covered by at least one cover element. The corresponding sealable opening of the assembly room may be covered by at least one corresponding cover element. The at least one cover element and the at least one corresponding cover element are configured to couple with each other to open jointly.

For example, the sealable opening and the corresponding sealable opening may be provided as sliding doors. The at least one cover element/corresponding cover element maybe a door leaf of the respective sliding door. Coupling the cover element(s) with the corresponding cover element(s) can be achieved e.g. by magnetic means, positive locking means, or any other coupling or a locking means. Preferably, magnets (permanent of electric) are distributed at least on an edge portion of the cover element(s)/corresponding cover element(s). This allows coupling the cover element(s) with corresponding cover element(s) in a defined manner. Thus, the non-sterile outer faces of the cover element(s)/corresponding cover element(s) cover each other after coupling. Thus, the risk for contaminating the assembled smart tank(s) when being transferred through the sealable openings from the assembly room to the clean room bag can be further reduced.

The cover element(s)/corresponding cover element(s) are movable with respect to the clean room bag and/or the assembly room. The cover element(s)/corresponding cover element(s) and the clean room bag /assembly room may be sealed, e.g. by at least one brush seal, a lip seal and/or the like. Even more preferably, the cover element(s)/corresponding cover element(s) maybe connected with the clean room bag, or the assembly room via a flexibel portion, such as a sealing foil portion. The flexibel portion may be connected to the cover element on a first side and to the clean room bag on a second side. Likewise, the flexibel portion maybe connected to the corresponding cover element on a first side and to the assembly room on a second side. Thus, the sealable opening can be opened/closed without providing a gap between the cover element and the clean room bag and/or the corresponding cover element and the assembly room, respectively. Thus, the contamination risk can be further reduced.

The clean room bag may further comprise a sealing frame or may be associated with a sealing frame. The sealing frame may be configured to frame the sealable opening of the mobile storage means and/or the corresponding sealable opening of the assembly room. Thus, an additional seal is provided that seals the opening from the environment, when being opened. Thus, the contamination risk can be further reduced. The sealing frame may be a separate frame, may be provided on the mobile storage means, and/or the assembly room.

The system may further comprise a UV-light source. The UV-light source may be associated with the sealable opening of the mobile storage means and/or the corresponding sealable opening of the assembly room. Thus, possible contaminants on the outer surface of the sealable opening can be removed/destroyed prior to and/or during opening the sealable openings. The at least one UV-light source may be provided within the sealing frame. Preferably, the UV-light source is configured to illuminate the sealable opening(s) from different orientations. Thus, contaminants can be removed/destroyed effectively.

The sealing frame may further comprise a port that allows evacuating a space between the sealing frame and the closed sealable openings of the mobile storage means and/or the assembly room prior to opening the sealable openings. Thus, particles and/or contaminants can be effectively removed, and the risk of contamination can be further reduced.

The clean room bag may comprise at least one rail for guiding at least one received assembled smart tank within the clean room bag. Thus, positioning the assembled smart tank(s) within the clean room bag is facilitated. For example, a first smart tank may be set on the rail. A second smart tank can be interconnected with the first smart tank and also set on the rail. Thereby, the first smart tank can be guided further into the clean room bag. Repeating this, an entire process line can be assembled and guided into the clean room bag.

The system may further comprise at least one height compensation means, that is adapted to be coupled to a received smart tank, and to adjust the height of the smart tank, so that an access plate element of the smart tank is installed at substantially the same height of a further smart tank received within the clean room bag.

The height compensation means may be adapted to be coupled to a first smart tank, so that the top plate element of the first smart tank, which may be an access plate element, is installed at substantially the same height as the top plate element of the second smart tank, when the height compensation means is coupled to the first smart tank. Thus, access points of the smart tank, such as ports of the smart tanks, filters of the smart tanks and/or actuating means of valves of the smart tanks can be accessed and operated at substantially the same height. Thereby automated actuation and operation of the smart tank(s) and the associated process line via at least one handling manipulator is facilitated.

The height compensation means may comprise a platform for receiving the smart tank and a stand. The stand may be variable in height. Thus, a height compensation means may be used with different kinds of smart tanks. For example, the stand may comprise a telescope mechanism or a folding mechanism. A stand that is variable in height may be actuatable, e.g. by an electric or hydraulic drive, so as to provide an automated height variation. Alternatively, the handling manipulator may adjust the height of the height compensation means. Further, the smart tank and/or the height compensation means may comprise at least one wheel. Said at least one wheel facilitates moving the smart tank to form e.g. a smart tank system comprising multiple smart tanks. The wheel may be associated with a brake. Thus, undesired movement of the smart tank can be prevented.

Further, a height compensation means may be provided underneath the clean room bag and may be part of a guide rail frame of the handling manipulator. The height compensation means may comprise multiple platforms that may be coupled to a smart tank within the clean room bag. The height of the platforms can then be adjusted separately, to provide the access plate elements of the smart tanks at substantially the same height.

The clean room bag may be initially stored in a frame assembly, i.e. prior to being installed for receiving smart tanks. The frame assembly may comprise air-permeable wall elements, wherein the clean room bag may comprise an air inlet port that serves for pressurizing the stored clean room bag for leakage testing. Thus, the clean room bag can be pressurized while being stored in the frame assembly and the change of pressure over time can be measured. Further, the port may be used for venting the clean room bag. As the frame assembly comprises air-permeable wall elements, air that leaves the stored clean room bag can also leave the frame assembly and thus, leakages can be detected. The air-permeable wall elements may be provided in form of wall elements with through holes, such as punctured sheets and/or in form of wall elements that are covered with an air-permeable means, such as an open-pored foam, a fabric, a non-woven, and/or the like. Particularly, leakages can be detected by comparing the measured change of pressure over time with a predefined threshold. In case the change of pressure over time exceeds the threshold, there is leakage and the assembly bag cannot be used.

The handling manipulator may comprise a manipulator head, wherein the manipulator head may be adapted to couple with the access plate element and/or the adaptor plate element, and wherein the manipulator head comprises at least one of the following:
- a cover element adapted to cover the adaptor plate at least partially, so that at least one access point of the adaptor plate is covered. Covering the access point reduces the risk of contamination.
- a UV-radiation means, the UV radiation means being adapted to irradiate at least one access point of the adaptor plate and/or the access plate element. Using UV radiation potential contaminants can be destroyed.
- a pump head, for providing a negative pressure in an area of a covered access point of the adaptor plate. Thus, the covered space can be evacuated and/or potential contaminants may be removed.
- a fluid line, for supplying and/or removing fluid from the smart tank via an access point of the adaptor plate. The fluid may be an operating fluid and/or an bio-chemical fluid. In particular, the fluid may be a buffer solution and/or an acid or a base for adjusting the pH value within the smart tank.
- a gas line, for supplying and/or removing gas from the smart tank, via an access point of the adaptor plate. The gas may be an operating gas, such as pressurized air. Thus, the smart tank can be pressurized for controlling the flow of bio-chemical medium stored in the tank. Further, O2, N2, and/or CO2 may be supplied via a gas line. The gases maybe supplied vie a common gas line or via separate gas lines. Further, the gas line may be associated with a flow meter, such as a gas flow meter, and/or a flow controller, such as a gas flow controller. Accordingly, the amount of gas being supplied/removed from the smart tank can be monitored and/or controlled.
- a valve actuating means, for actuating a valve of the smart tank via an access point of the adaptor plate. The valve actuating means may be part of a drive mechanism and may be adapted to couple to an actuating means of a valve of the smart tank. Thus, the valve can be controlled (e.g. opened/closed), using the handling manipulator.
- a driving means for driving an operating means of the smart tank via an access point of the adaptor plate. The operating means may be a stirring means. Thus, e.g. the stirring of the smart tank can be controlled, using the handling manipulator.
- a sensor for measuring operating conditions of the smart tank, such as a pressure sensor. This allows e.g. for integrity or leakage testing.
- a spectrography means, and/or
- any other kind of sensor.

The handling manipulator may comprise at least one interchangeable manipulator head. A first interchangeable manipulator head may provide different functionality than a second interchangeable manipulator head. Different functionality may be provided by providing the respective manipulator head with different combinations of the above-mentioned means that may be part of a manipulator head. For example, a first manipulator head may comprise a drive mechanism including a valve actuating means and/or a driving means. A second manipulator head may comprise a fluid and/or a gas line for supplying operating and/or bio-chemical medium to and/or removing from the smart tank.

Further, the handling manipulator may be adapted to weld a foil portion, that is sandwiched between the adaptor plate element and the access plate element with the adaptor plate element and/or the access plate element. Accordingly, the handling manipulator may comprise a foil welding device. Welding the foil portion to the adaptor plate element and/or the access plate element allows to provide a tightly sealed connection and thus prevents entry of contaminants. Further, the handling manipulator may be adapted to weld and/or seal a hose portion of the smart tank. Thus, a tightly sealed hose connection can be provided.

The handling manipulator may further be adapted for sampling. The system and/or the handling manipulator (particularly the manipulator head) may comprise a sampling means. The sampling means is adapted to be coupled to a sampling channel of the smart tank for sampling from the smart tank. The sampling means may be cylinder, a cartridge and/or a syringe. The sample maybe transferred to the sampling means by applying a positive pressure to the smart tank and/or by applying a negative pressure within the sampling means. Prior to sampling an optional valve that is associated with the sampling channel may be opened, preferably by the handling manipulator.

The sampling means may be adapted to be guided along multiple interconnected smart tanks. For example, a guide rail maybe provided that guides the sampling means. The guide rail may be integrally formed in sidewall elements of the smart tanks. Further, the sampling means may be guided by the handling manipulator. The sampling means may be adapted to be coupled to a sampling channel of any one of the interconnected smart tanks for sampling from the respective smart tank. Thus, samples can be taken from different smart tanks.

Particularly, the handling manipulator may be adapted to move and/or actuate the sampling means.

The system may further comprise a sampling means magazine, for receiving multiple sampling means. The sampling means magazine may be adapted to output a filled sampling means after sampling. Optionally, the sampling means magazine outputs the filled sampling means to an air lock of the clean room bag, to a cooled storing device, such as a refrigerator, to an analyzing means, such as an add line analyzer, and/or the like. Thus, the sample can be taken under clean room/sterile environment and can be transferred to the outside of the clean room bag.

The sampling magazine may be adapted to be guided along multiple interconnected smart tanks. For example, a guide rail may be provided that guides the sampling magazine. After a sampling means is filled and output the sampling magazine may provide a further sampling means for taking a further sample.

The system may further comprise a control unit. The control unit maybe adapted to receive sensor signals from at least one sensor of the at least one smart tank and to control the at least one handling manipulator based on the received sensor signals to control the at least smart tank according to a predetermined control scheme. The sensor may be any kind of sensor. For example, the sensor may be a temperature sensor, a pH sensor, a volume sensor, a balance, and/or the like. The handling manipulator may then open/close valves to control a flow of medium, and/or directly provide medium to the tank, in order to adjust temperature, pH, volume, weight and/or the like.

The object is further achieved by a handling manipulator, as described above and/or by a method for automatically operating at least one smart tank in a bio-pharma process line. The method comprises the steps of providing a system, as described above, receiving sensor signals from at least one sensor of the at least one smart tank, controlling at least one handling manipulator based on the received sensor signals and operating the at least smart tank according to a predetermined control scheme, using the handling manipulator.

### Brief Description of the Figures

In the following, the accompanying figures, that schematically show embodiments of the invention are described. Here,
- Fig. 1: schematically shows an exploded view of a smart tank;
- Fig. 2A: schematically shows a smart tank in an assembled state;
- Fig. 2B: schematically shows a further smart tank in an assembled state;
- Fig. 3: schematically shows a valve comprising an actuating rod;
- Fig. 4A: schematically shows a top plate element, comprising a valve;
- Fig. 4B: schematically shows a further top plate element;
- Fig. 5: schematically shows a smart tank system;
- Fig. 6: schematically shows a further smart tank system;
- Fig. 7: schematically shows smart tanks, having different volumes;
- Fig. 8: schematically shows a clean room bag;
- Fig. 9: schematically shows further clean room bag(s);
- Fig. 10A: schematically shows inlet openings of a clean room bag;
- Fig. 10B: schematically shows a sealable opening in an open state;
- Fig. 10C: schematically shows a sealable opening in a closed state;
- Fig. 11A: schematically shows an adaptor plate element prior to assembly;
- Fig. 11B: schematically shows an assembled adaptor plate element;
- Figs. 12A to C: schematically show assembling steps of an adaptor plate element;
- Fig. 13: schematically shows a handling manipulator;
- Fig. 14A: schematically shows a handling manipulator operating a smart tank;
- Fig. 14B: schematically shows a handling manipulator operating a smart tank;
- Fig. 15: schematically shows a handling manipulator operating a smart tank;
- Fig. 16: schematically shows a perspective view of a manipulator head;
- Fig. 17A: schematically shows a perspective view of a manipulator head;
- Fig. 17B: schematically shows a bottom view of the manipulator head;
- Fig. 18A: schematically shows a sampling means;
- Fig. 18B: schematically shows an operating principle of a sampling means and a sampling means magazine;
- Fig. 19: schematically shows a clean room bag in an initial state, and
- Fig. 20: schematically shows a flow diagram of a method.

### Detailed description of the Figures

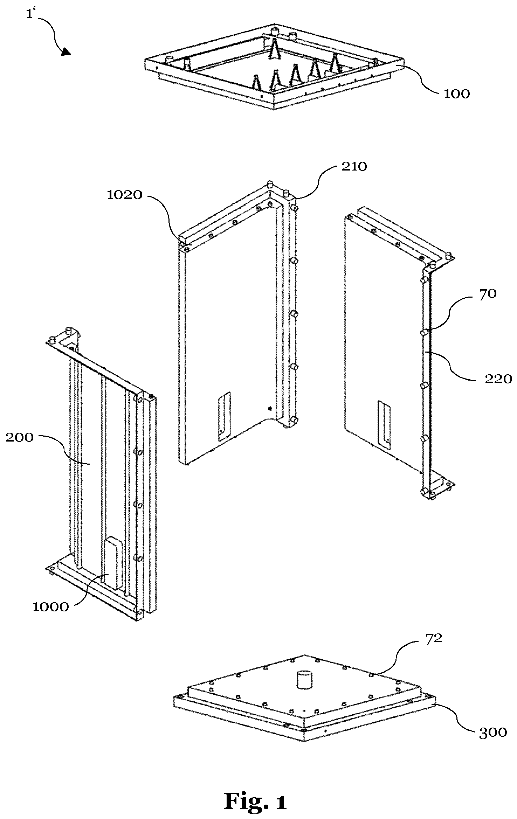

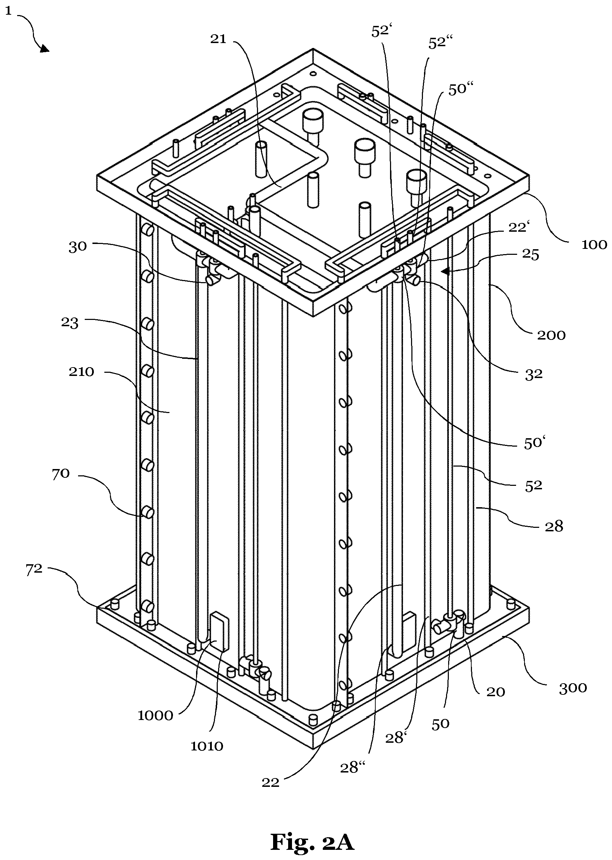

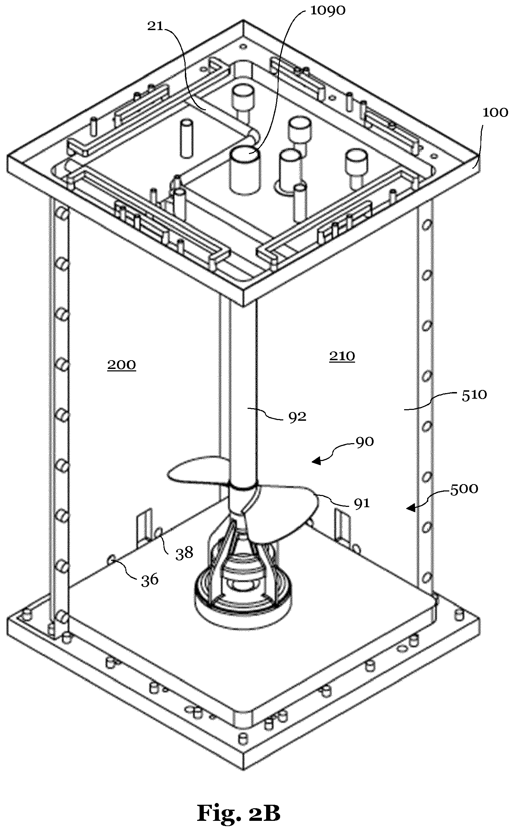

In particular, Fig. 1 schematically shows an exploded view of a smart tank 1. The smart single elements of the smart tank can be assembled to a smart tank 1 (as e.g. shown in Figs. 2A, 2B). The assembly may be automated or manually. The smart tank 1 comprises a top plate element 100, at least one sidewall element 200, 210, 220, and a bottom plate element 300. As depicted, the smart tank of Fig. 1 comprises three sidewall elements 200, 210, 220.

The top plate element 100, the sidewall elements 200, 210, 220 and the bottom plate element 300 can be assembled to form a reservoir 500 for receiving at least one biochemical and/or operating medium. The channel may be associated with a valve that allows to open/close the channel. The valve may also be a flow control valve, that allows to control the flow through the channel. The channel may further comprise a port, such as an inlet and/or outlet port.

At least one of the top plate element 100, the sidewall elements 200, 210, 220 and the bottom plate element 300 comprises at least one channel, for guiding the at least one biochemical medium and/or an operating medium. The at least one channel may be associated with a valve, wherein the valve allows opening and/or closing the channel. Further, the valve may be a flow control valve that allows to control or set a desired flow rate through the channel.

The smart tank may comprise at least one sealing member 1020 for providing a sealed connection between the elements (top plate element 100, sidewall elements 200, 210, 220 and bottom plate element 300). The sealing member 1020 may be arranged circumferentially at each sidewall element 200, 210, 220, top plate element 100 and/or bottom plate element 300. When the elements are assembled to form the reservoir 500, the sealing member 1020 may be compressed, so as to provide a retaining force that acts on assembly-connecting means 70 and corresponding assembly-connecting means 72. Thereby, a self-retaining engagement may be provided.

Each of the elements (top plate element 100, sidewall elements 200, 210, 220 and bottom plate element 300) may be adapted to be provided with a sensor 1010 and/or a sensor module 1000. A sensor module 1000 may comprise multiple sensors, such as at least one of a pH sensor, a temperature sensor, a dissolved oxygen sensor, a biomass sensor, a foam sensor, a pressure sensor, a flow sensor, an O2 sensor, a N2 sensor, a CO2 sensor, and spectroscopy means, such as RAMAN, NIR and/or UV spectroscopy means. The sensor module may be connectable to the respective top plate element 100, sidewall element 200, 210, 220 and/or bottom plate element 300. The sensor module 1000 maybe provided with a power source such as a rechargeable battery, that allows to operate the sensor module 1000 autonomously. Further, the sensor module 1000 may comprise a data interface, particularly a wireless data interface for transferring the measured sensor data to a respective control or storing unit.

Fig. 2A shows a smart tank 1 in an assembled state. This smart tank may serve for storing and/or transporting a biochemical medium or may be a bio reactor in a bio-pharma process line. This smart tank can be operated by a handling robot, as will be described in greater detail below. The shown smart tank comprises a top plate element 100, multiple sidewall elements 200, 210, and a bottom plate element 300. The top plate element, the sidewall elements and the bottom plate element are arranged to form a reservoir for receiving at least one biochemical medium.

The smart tank 1 comprises further channels 20, 21, 22, 23 for guiding the at least one biochemical medium and/or an operating medium. The channels extend within at least one of the top plate element 100, the sidewall elements 200, 210 and/or the bottom plate element 300.

For example channel 21 extends in the sidewall element 200 and the top plate element 100. Other channels may be provided that extend in the at least one sidewall element and at least one of the top plate element and the bottom plate element. Each of the channels may be at least one of the following channel types: An inlet channel, for guiding a biochemical medium and/or an operating medium to the reservoir of the smart tank. An outlet channel, for removing a biochemical medium and/or an operating medium from the reservoir of the smart tank. A retentate channel, for transferring a retentate back into the smart tank or out of the smart tank. A bypass-channel, for guiding a biochemical medium and/or an operating medium, wherein the bypass-channel is not connected to the reservoir of the smart tank. Alternatively, the bypass-channel can be adapted to be fluidically separated from the reservoir of the smart tank, e.g. by means of a valve. A heating or cooling channel for guiding a tempered heating or cooling medium. A sampling channel, for taking a sample of biochemical medium and/or of operating medium from the reservoir of the smart tank. Particularly, the smart tank may comprise multiple channels of different channel-types and/or the same channel type.

In the smart tank shown in Fig. 2A, channel 20 serves as outlet channel, particularly for removing waste. Channel 22 can be either an inlet or an outlet channel. This channel 22 enters the reservoir at a bottom side of the smart tank (i.e. near the bottom plate element). Channel 22' can also be either an inlet or an outlet channel. This channel 22 enters the reservoir at a top side of the smart tank (i.e. near the top plate element).

Channel 21 is a bypass-channel, that allows guiding a biochemical medium and/or an operating medium, via the smart tank, without entering the reservoir of the smart tank. Channels 28, 28' and 28" are not connected to the reservoir and may serve as cooling and/or heating channels.

Channels 21, 22 and 22' meet each other at a channel junction 25. This channel junction is provided with at least one (in the embodiment shown with two) valves 50', 50". These valves 50', 50" are actuatable from the outside of the smart tank, by means of an actuating means 52', 52". The actuating means 52', 52" are provided in form of actuation rods. Channel 20 is associated with a respective valve 50 which can be actuated from the outside of the smart tank, by means of actuating means 52. Each of the actuating rods 52', 52" /actuating means 52 can be actuated by the handling manipulator.

Further, the smart tank 1 comprises ports 30, 32. The ports are each associated with respective channel(s). The ports may be chosen from a group of port-types, comprising the following port-types: a fluid inlet port, a gas inlet port, a fluid outlet port, a gas outlet port, a cell blead port, a medium supply port, a medium remove port, an element-interconnecting port, and a tank-interconnecting port.

For example, port 32 is associated via valves 52', 52" with channels 21, 22 and 22'. Accordingly, this port 32 may serve as fluid inlet port, gas inlet port, fluid outlet port, gas outlet port, medium supply port, medium remove port, or tank-interconnecting port. Chanel 20, which serves as outlet channel is associated with an outlet port (not shown) on a bottom side of the bottom plate element 300. Thus, medium can be transferred to and/or out of the smart tank via the ports, e.g. by using a handling manipulator.

Particularly, all sides of the smart tank (or at least some of the sides) may provide the same port interface. I.e. ports are arranged at the same position. Thus, a smart tank can be easily interconnected to a further smart tank. Further, all side wall elements of a smart tank may be identically structured. Thus, the number of different elements required for setting up a smart tank is reduced.

Fig. 2 Bschematically shows a further smart tank that can be operated by a handling manipulator. This smart tank may serve for transporting and storing a biochemical medium. The smart tank comprises a stirring means 90. The smart tank shown in Fig. 2B corresponds to the smart tank described with respect to Fig. 2A, wherein two sidewall elements are not depicted, to allow a view inside the reservoir 500 of the smart tank. The inner surface 510 of the reservoir maybe coated e.g. by a glass coating.

The stirring means 90 comprises at least one stirring member 91, wherein the stirring member comprises multiple stirring blades. Further, the stirring means comprises an actuating rod 92. Said actuating rod 92 is supported and sealed in the top plate element 100 of the smart tank. The actuating rod is engageable with a drive mechanism (not shown), such as an electric drive mechanism, provided on the outside of the smart tank. The drive mechanism maybe part of a handling manipulator (not shown) that allows automated control of the smart tank and/or a smart tank system.

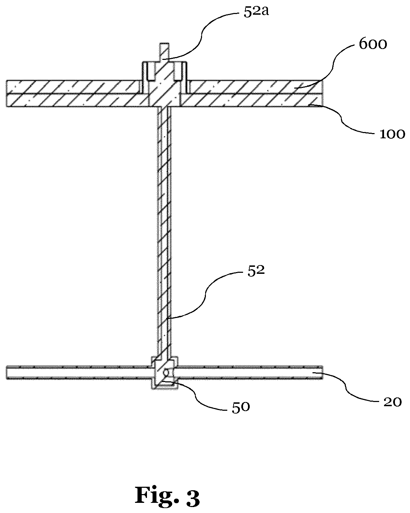

Fig. 3 schematically shows a valve 50 comprising an actuating rod 52. The valve 50 is associated with a channel 20. The valve 50 is coupled to an actuating rod 52, which has at its end a coupling portion 52a. The actuating rod 52 is supported in the top plate element 100 and/or the adaptor plate element 600 and can be easily installed. Preferably, the actuating rod 52 can be slided into the sidewall element/top plate element. The handling manipulator, particularly the handling manipulator head, may comprise a valve actuating means (not shown) to couple with the coupling portion 52a of actuating rod 52. By actuating (rotating) the valve actuating means, the handling manipulator can control the valve 50. The valve 50 may comprise a sealing member, that allows to seal the valve 50 and the channel 20, so that medium cannot flow along the actuating rod 52. The sealing member may be integrally formed (e.g. using 2K-injection moulding) and/or maybe assembled. Further, the sealing member may be associated with the smart tank (e.g. the side wall element) and/or with the actuating rod 52.

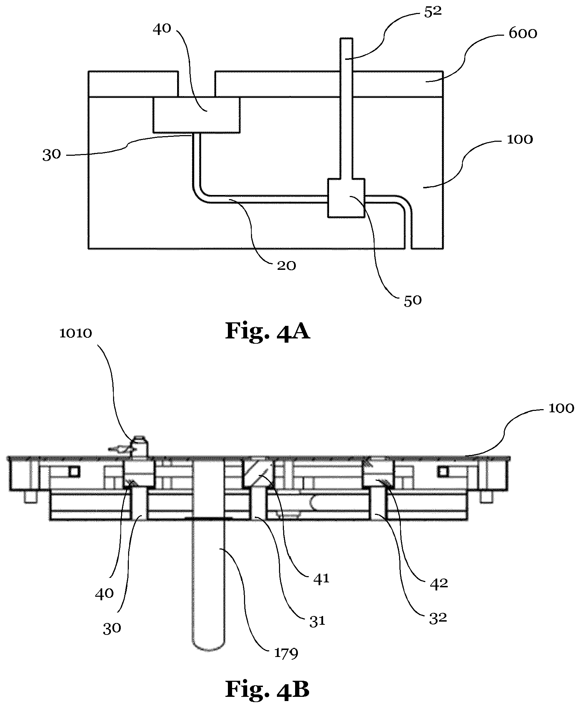

Fig. 4A schematically shows a top plate element 100 that comprises a valve 50 for opening/closing channel 20. The valve 50 is a mechanical valve that is configured to be actuatable from the outside of the smart tank, by means of an actuating means 52. The actuating means 52 is provided in form of an actuation rod that can be actuated from the outside of the smart tank, e.g. by a handling manipulator (not shown). For opening/closing the channel, the actuating means 52 can be rotated or axially displaced, depending on the type of associated valve. The actuating means may be supported in an adaptor plate element 600 that can be installed on top of the top plate. The adaptor plate element may cover a filter 40 and/or a port 30 at least partially. Thus, operating and/or biochemical medium can be guided to the smart tank via the filter 40. The operating and/or biochemical medium may be provided by the handling manipulator. Further, the handling manipulator may be adapted to remove operating and/or biochemical medium from the smart tank via port 30 and optionally filter 40.

Fig. 4B schematically shows a further top plate element 100 that comprises multiple filters 40, 41, 42 that cover associated ports 30, 31, 32. Further, a pressure sensor 1010 maybe provided. The filter covered port 30 and the pressure sensor 1010 allow for integrity testing the smart tank (including all channels and tank interconnections) and single filters. Further, an inlet 179 may be provided that allows to insert a spectrography means 17900 of a handling manipulator into the smart tank.

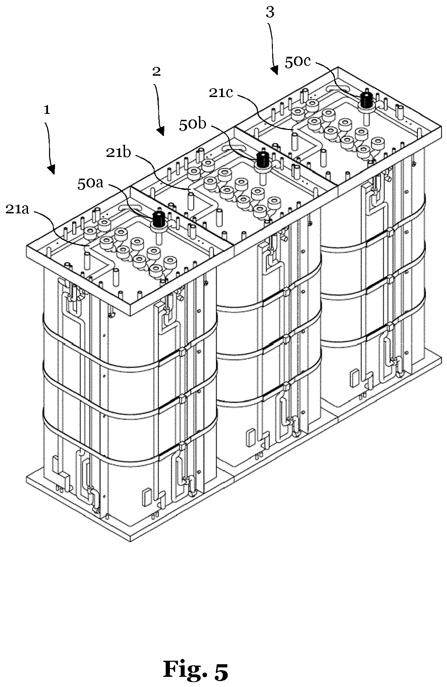

Fig. 5 schematically shows a smart tank system that comprises multiple smart tanks 1, 2, 3. The smart tanks are directly interconnected with each other. At least one of the one or more channels 21a of the first smart tank 1 is fluidically connected to a respective channel 21b of the second smart tank 2 which can be connected to a respective channel 21c of the third smart tank. Depending on the position of the valves 50a, 50b and/or 50c, which can be operated using a handling manipulator, medium can be e.g. transferred from the first smart tank to the second smart tank or the third smart tank. When transferring medium to the third smart tank medium can either be guided through the reservoir of the second smart tank or medium can bypass the reservoir of the second smart tank. Thus, a bio-pharma process of a bio-pharma process line that includes the smart tank system can controlled by operating the valves.

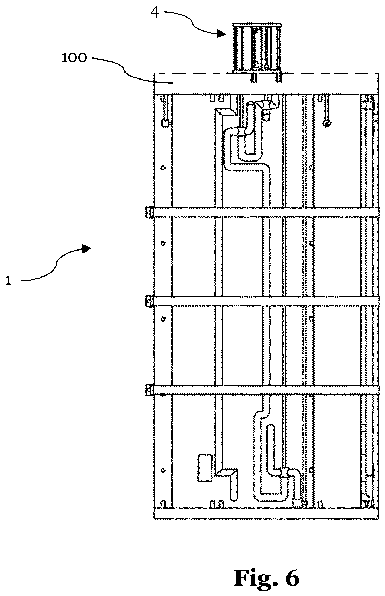

Fig. 6 schematically shows a further smart tank system. The smart tank system comprises two smart tanks, a larger smart tank 1 and a smaller small tank 4. The smaller smart tank 4 is provided on top of the larger smart tank 1. The smaller smart tank 4 can be provided on top of the larger smart tank 1 by using the handling manipulator (not shown). The handling manipulator may comprise a gripping device that is adapted to grip the smaller smart tank and to put it on top of the larger smart tank 1. Both smart tanks may be fluidically connected, via a channel or port (not shown). The channel or port may comprise a sterile filter, allowing to transfer medium from the smaller smart tank to the larger smart tank without contaminating the larger smart tank. The sterile filter may be provided in the top plate element of the larger smart tank 1. The fluid connection may be controlled by a valve that is preferably operable by at least one handling manipulator. The smaller smart tank may serve to provide an operating medium and/or a biochemical medium to the larger smart tank. As the smaller smart tank 4 is installed on top of the larger smart tank 1, it is possible to transfer medium from the smaller smart tank to the larger smart tank by using gravitation. The medium maybe gaseous, fluidic and/or solid (e.g. in form of a powder, crystals, granulate, and/or the like). For transferring a solid medium into the larger smart tank 1, a vibrating means may be provided (e.g. at the handling manipulator) that allows to control a dose of solid medium, supplied to the larger smart tank. Further, in particular for transferring fluidic and/or solid medium pressure (positive or negative) may be applied to the smaller and/or larger smart tank.

Further, the smaller smart tank may be provided outside the clean room bag, wherein the larger smart tank may be provided within the clean room bag. In other words, a foil portion of the clean room bag may be sandwiched between the smaller smart tank 4 and the larger smart tank 1. The foil portion may be pierced/removed, as described above, using the handling manipulator. Further, the foil portion may be welded to the top plate of the smart tank 1, an adaptor plate element and/or to the smaller smart tank 4.

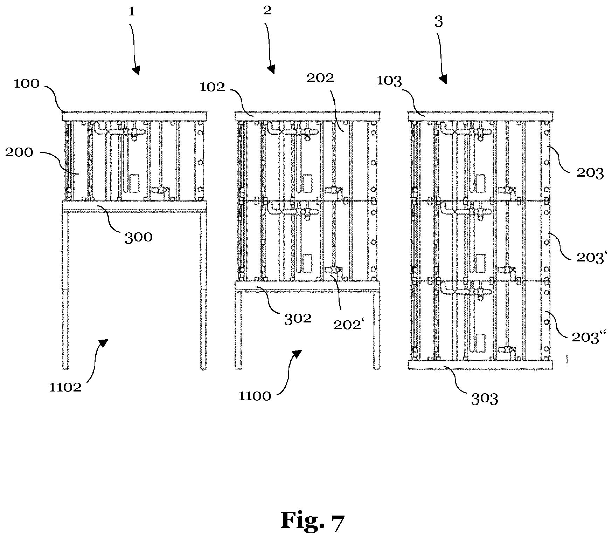

Fig. 7 schematically shows smart tanks 1, 2, 3, having different volumes. To provide different volumes, it is possible to provide different kinds of sidewall elements. Thus, different volumes can be provided, using the same top plate element and bottom plate elements. Further, different volumes can be provided by stacking multiple sidewall elements 202, 202'; 203, 203', 203". As shown in Fig. 7, smart tank 1 comprises sidewall elements 200 that are all arranged in the same level of the smart tank 1. This smart tank has the following stack of elements: bottom plate element 300 / side wall element 200 / top plate element 100.

Smart tanks 2 and 3 comprises multiple sidewall elements 202, 202'; 203, 203', 203", wherein the groups of side wall elements are arranged in different levels of the smart tank. Smart tank 2 has the following element stack: bottom plate element 302 /side wall element 202' / sidewall element 202 / top plate element 102 and smart tank 3 has the following element stack: bottom plate element 303 / side wall element 203" / side wall element 203' / sidewall element 203 / top plate element 103.

As described above, channel portions extending in the respective sidewall elements are interconnected with corresponding channel portions in the neighboring element (sidewall element, bottom plate element or top plate element). Likewise, actuating means for actuating a valve and/or for driving a stirring means and/or the like that are provided in the sidewall element(s) may be coupled to corresponding actuating means of a neighboring element. Thus, a valve or the like provided e.g. in sidewall element 202' or 203" can be actuated from the top side of the smart tank using the handling manipulator.

As shown, the height dimension of the first smart tank 1 is smaller than a height dimension of the second smart tank 2 and the third smart tank 3. To provide the top plate elements 100, 102 and 103 on substantially the same height, a height compensation means 1100, 1102 can be provided. Said height compensation means 1100, 1102 is adapted to be coupled to the smart tank 1, 2 and allows to install the top plate element 100 of the first smart tank 1, the top plate element 102 of the second smart tank 2 and in substantially the same height as the top plate element 103 of the third smart tank 3. Thus, interconnection of the smart tanks as well as automated operation of the smart tanks is facilitated.

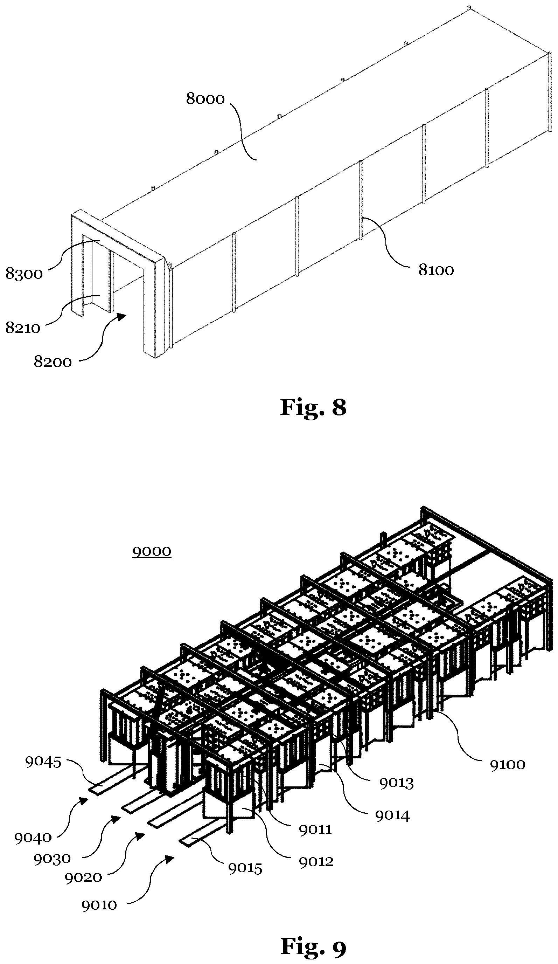

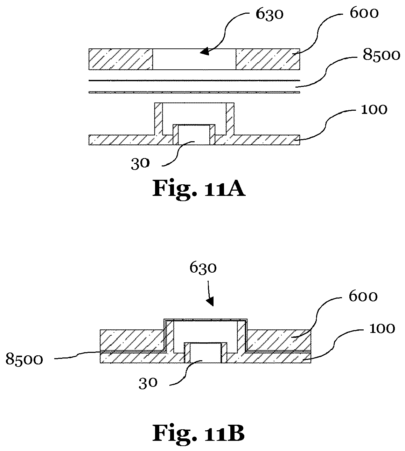

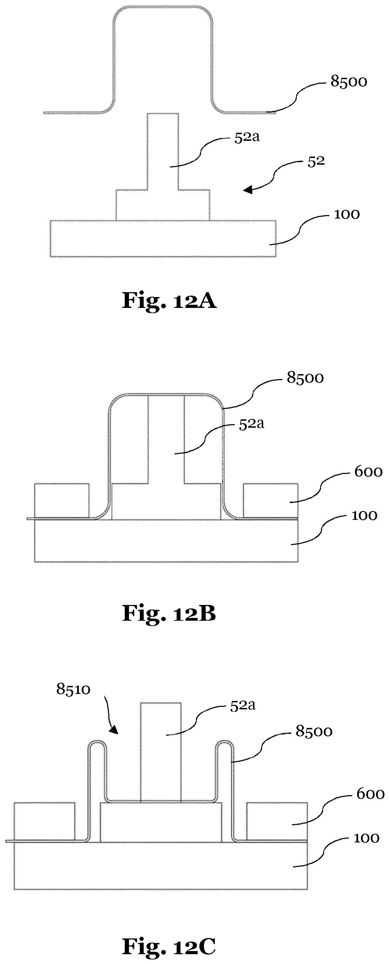

Fig. 8 schematically shows a clean room bag 8000 that is configured to receive at least one smart tank (cf. Fig. 9) and to provide a clean room environment or even a sterile environment for the at least one smart tank when the at least one smart tank is received in the clean room bag 8000. The clean room bag 8000 comprises at least one foil portion, that is adapted to be sandwiched between an access plate element of the smart tank and an adaptor plate element, so as to define at least one access point to provide access to the smart tank from the outside of the clean room bag, via the access plate element (cf. Figs. 11A, 11B and Figs. 12A to 12C). The clean room bag 8000 can sealingly couple to an assembly room (not shown) that servers for assembling at least one smart tank, wherein the clean room bag is configured to receive the at least one assembled smart tank from the assembly room.

The clean room bag 8000, 9000 is held by a support structure 8100, 9100. The support structure may be connected or integrally formed with the guide rails of the handling manipulator. The support structure 8100, 9100 maybe provided in form of multiple U-shaped frames. However, other support structures can be used. The support structure 8100, 9100 may be formed integrally with the clean room bag 8000, 9000, or may be a separate support structure that can be coupled with the clean room bag. The support structure 8100, 9100 is used to span the clean room bag and thus to open up the operating space.

For receiving smart tanks, the clean room bag 8000 includes at least one sealable opening 8200. The sealable opening 8200 of the clean room bag is configured to open jointly with a corresponding sealable opening of an assembly room (not shown), when the clean room bag is sealingly coupled to the assembly room. Particularly, the sealable opening 8200 of the clean room bag 8000 may be a sliding door and/or may be covered by at least one cover element 8210. The at least one cover element 8210 may be adapted to couple with a corresponding cover element of a corresponding sealable opening of an assembly room (not shown) that is covered by the at least one corresponding cover element, to open jointly with the corresponding cover element. The cover element 8210 may comprise a magnetic means for coupling with a corresponding cover element. Additionally, or alternatively, the coupling may be achieved by form fit or any other coupling means.

Further a sealing frame 8300 may be provided. The sealing frame 8300 may be configured to frame the at least one sealable opening 8200 of the clean room bag 8000, wherein the sealing frame 8300 may further comprise a UV-light source (not shown), that is associated with the sealable opening 8200 of the clean room bag 8000. Alternatively, or additionally, the sealable frame can be evacuated. Thus, contaminants on the outside of the sealable opening 8200 can be removed and/or destroyed prior to opening the sealable opening 8200.

Fig. 9 schematically shows a further clean room bag 9000. The clean room bag 9000 is adapted to receive smart tanks 9011, 9013 in multiple rows 9010, 9020, 9030, 9040. Here, four rows 9010, 9020, 9030, 9040 of smart tanks are shown. However, the clean room bag 9000 may be adapted to receive at least one row of smart tanks, preferably at least two rows of smart tanks, even more preferably at least three rows of smart tanks, even more preferably at least four rows of smart tanks, even more preferably at least five rows of smart tanks, and most preferably at least eight rows of smart tanks. The smart tanks of a row may be interconnected and/or the smart tanks of different rows may be interconnected so that a bio-chemical medium and/or operating medium may be transferred from a smart tank to another smart tank within a row and/or from a smart tank to another smart tank of different rows. For example, a first row 9020 may include tanks of a bio-pharma process line that outputs the product to be manufactured. A second row 9010 and/or third row 9030 may provide additives or further educts for producing the product (e.g. operating medium, such as a buffer solution). Further, the rows of smart tanks may be interconnected in a way that different products can be manufactured starting from a single educt.

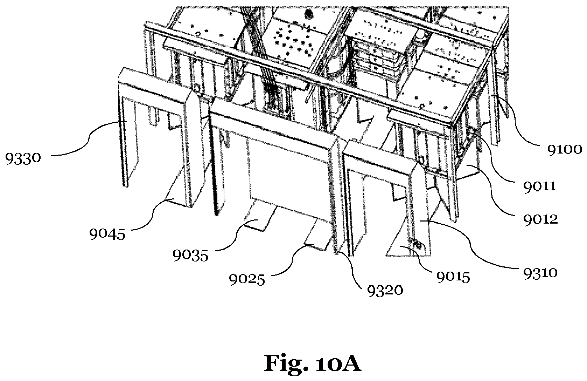

The clean room bag 8000, 9000 may comprise at least one rail 9015, 9025, 9035, 9045 for guiding at least one received assembled smart tank within the clean room bag (cf. also Fig. 10A). Each of the rails 9015, 9025, 9035, 9045 may be associated with a row of smart tanks.

The clean room bag, at least one of the received smart tanks and/or the system may further comprise at least one height compensation means 9012, 9014, that may be coupled to a smart tank 9011, 9012, and to adjust the height of the smart tank 9011, 9012, so that an access plate element of the smart tank 9011, 9012 is installed at substantially the same height of a further smart tank 9011, 9012 received within the clean room bag 8000, 9000. Thus, automated operation of the smart tanks can be facilitated.

Fig. 10A schematically shows sealable openings of the clean room bag 9000. For receiving smart tanks, the clean room bag 9000 includes three sealable openings, each of the openings being associated with a sealable frame 9310, 9320, 9330. Alternatively, to providing a clean room bag for receiving multiple rows of smart tanks, multiple clean room bags can be provided, wherein each of the clean room bags may receive at least one row of smart tanks. The rows of smart tanks of different clean room bags may be interconnected, so that bio-chemical and/or operating medium can be transferred from one row to another row. The interconnection of the smart tanks, e.g. of different rows, may be achieved by at least one hose-based connection. The hose-based connection may be installed outside the smart tank, wherein a respective hose is coupled via respective access plate elements and/or adaptor plate elements with the respective smart tanks. The hose-based connection may be established, using the handling manipulator, i.e. automatically.

Generally, the handling manipulator may comprise a hose connection means, that is adapted to provide a hose and/or to connect a hose to the respective access plate element and/or adaptor plate element, sealingly. The hose connection may be achieved using a quick connector. Preferably, the hose may be welded to the respective access plate element and/or adaptor plate element. Further, the hose connection means may be covered by a cover element that may be provided with a UV-radiation means and or a pump head.

If different clean room bags are used, each clean room bag may provide the same or a different clean room class. Preferably, all clean room bags provide a sterile environment. Alternatively, in case of different clean room classes, for example, the actual product line may be provided in a sterile clean room bag, wherein operating media containing tanks (such as buffer solution tanks) may be provided in a clean room bag having a lower clean room class.

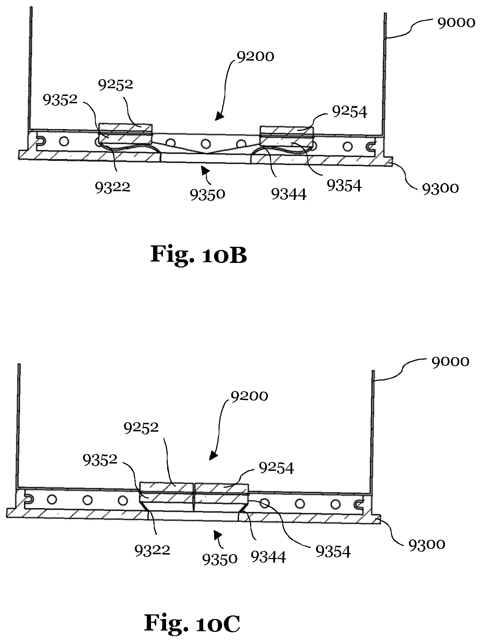

Fig. 10B and 10C schematically shows a sealable opening being coupled to a corresponding sealable opening. Fig. 10B shows an open state, wherein Fig. 10C shows a closed state. The sealable opening 9200 may be part of a clean room bag. The corresponding sealable opening 9350 is part of an assembly room 9300, preferably of an assembly bag. In the following the opening/closing is described.

The sealable opening 9200 allows receiving/removing smart tanks. An assembly room 9300 comprises at least one corresponding sealable opening 9350 that is configured for transferring smart tanks from the assembly room to the clean room bag. The sealable opening 9350 of the assembly room 9300 and the corresponding sealable opening 9200 of the clean room bag 9000 are configured to open jointly, when the assembly room 9300 is sealingly coupled to the clean room bag 9000.

Further, the sealable opening 9350 of the assembly room 9300 may be covered by at least one cover element 9352, 9354, such as a door leaf of a sliding door. The corresponding sealable opening 9200 of the clean room bag 9000 may be covered by at least one corresponding cover element 9252, 9254. The at least one cover element 9352, 7354 and the at least one corresponding cover element 9252, 9254 are configured to couple with each other to open jointly. The coupling can e.g. be achieved magnetically or by positive locking.

Figs. 11A and 11B schematically show an adaptor plate element 600, wherein Fig. 11A shows the adaptor plate element 600 prior to assembly and Fig. 11B shows an assembled adaptor plate element 600. The adaptor plate element 600 may be associated with at least one smart tank (not shown). Particularly the adaptor plate element may be associated with a single smart tank or with multiple smart tanks. The adaptor plate element 600 can be installed on the at least one smart tank, so that one of the at least one foil portions 8500 of the clean room bag is sandwiched between the access plate element 100 (e.g. a top plate element) of the smart tank and the adaptor plate element 600.

The adaptor plate element 600 defines at least one access point 630 to provide access to the smart tank from the outside of the clean room bag, via the access plate element 100. Accordingly, the smart tank can be provided in a clean room or sterile environment (e.g. within a clean room bag, cf. Figs. 8, 9, 10) and at the same time can be accessed from the outside of the clean room bag. Thus, a handling manipulator and other control or operating devices can be provided outside of the clean room bag.

The adaptor plate element 600 may cover a filter (not shown) and/or a port 30 of the smart tank at least partially. Thus, medium (bio-chemical and/or operating medium) can be transferred to and/or removed from the smart tank from the outside of the clean room bag.

The foil portion 8500 of the clean room bag, that is sandwiched between the access plate element 100 of the smart tank and the adaptor plate element 600, may be provided removably and/or pierceable in an area of the filter and/or port 30 of the smart tank. In Fig. 11B, the foil portion 8500 is pierceable. Thus, for accessing the smart tank from the outside of the clean room bag, a handling manipulator may pierce the foil portion 8500 covering port 30 for supplying/removing medium to/from the smart tank.

For accessing a smart tank from the outside of a clean room bag, the handling manipulator may carry out the following steps:
Step 1: Install an adaptor plate element 600 on the at least one smart tank, so that one of the at least one foil portions 8500 of the clean room bag is sandwiched between the access plate element 100 of the smart tank and the adaptor plate element 600. Optionally, remove a cover element, such as a cap, from a through hole (access point) of the adaptor plate element.
Step 2: Perforate the at least one foil portion 8500 of the clean room bag with a perforating means, such as a knife, needle, laser and/or the like. The perforation may be carried out in a way, that a foil portion is separated from the clean room bag.
Step 3: Optionally, gripping the separated foil portion 8500 using a gripping means, such as a vacuum gripper of the handling manipulator.
Step 4: Optionally, welding the remaining foil portion 8500 with the adaptor plate element 600 and/or the top plate element 100, preferably by using the handling manipulator.
Step 5: Optionally, placing a connector means, such as a quick connector means on the adaptor plate in an area of the removed/perforated foil portion.

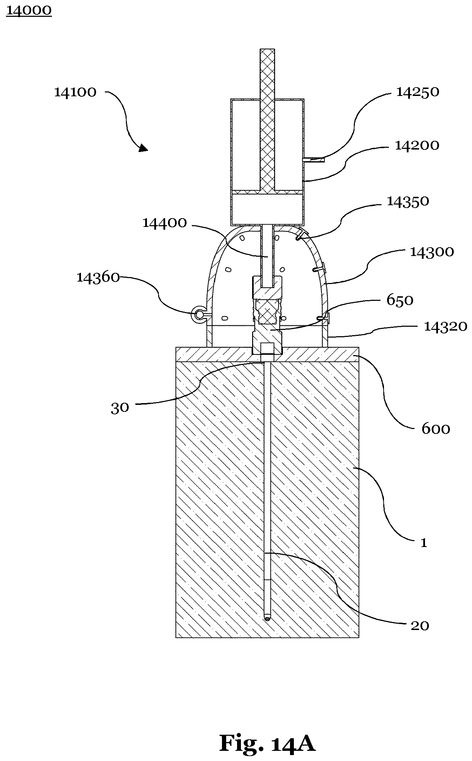

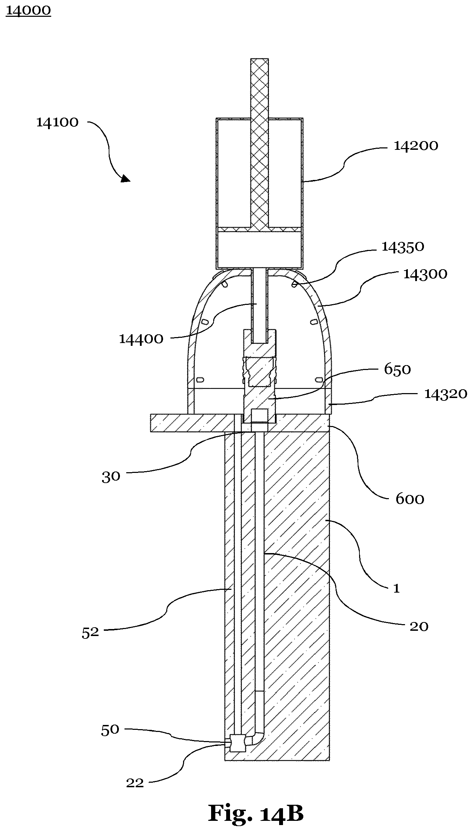

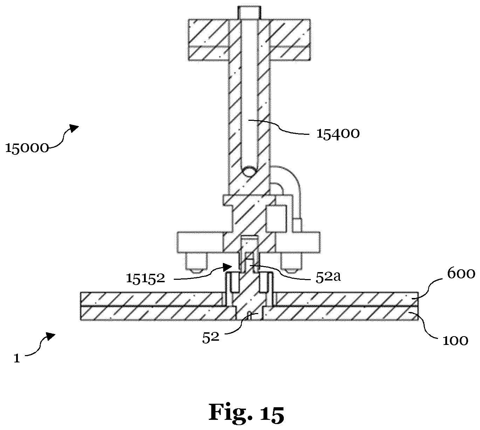

For facilitating access to the smart tank, a connector element may be provided. The connector element 650, such as a fluidical quick connector, may be inserted into the access point 300, preferably by the handling manipulator (cf. Figs. 14, 15).

Figs. 12A to C schematically show assembling steps of an adaptor plate element 600. Particularly, an access plate element, which may be a top plate element 100 of a smart tank, is shown. The top plate element supports an actuating means 52, comprising an actuating rod 52 of a valve (not shown). Said valve is coupled to the actuating rod 52, which has at its end a coupling portion 52a. The actuating rod 52 is supported in the top plate element 100 and/or the adaptor plate element 600. A handling manipulator, particularly a handling manipulator head, may comprise a valve actuating means (not shown) to couple with the coupling portion 52a of actuating rod 52. By actuating (rotating) the valve actuating means 52, the handling manipulator can control the valve 50.

A foil portion 8500 of a clean room bag may be provided removably and/or pierceable in an area of the actuating means 52. Alternatively, the foil portion 8500 maybe dimensioned so that operating the actuating means is possible, without removing/piercing the foil portion 8500.

The adaptor plate element 600 may comprise an access point 630 in form of a through hole. The adaptor plate element 600 may be installed on top of the access plate element 100 so that at least the coupling portion 52a of the actuating rod 52 extends out of the through hole of the adaptor plate element 600. The foil portion 8500 may cover the coupling portion 52a and thus, the smart tank can be provided entirely in a clean room/ sterile environment and can be operated from the outside of the clean room bag. Optionally, the foil portion 8500 maybe welded with the adaptor plate element 600 and/or the access plate element 100 in a welding zone 8510. Accordingly, the handling manipulator may comprise a foil welding device. Welding the foil portion 8500 to the adaptor plate element 600 and/or the access plate element 100 allows to provide a tightly sealed connection and thus prevents entry of contaminants. The welding device of the handling manipulator may be any suitable plastics welding device, such as hot plate welding, electro fusion welding, high frequency welding, laser transmission welding, circular welding, rotary friction welding, ultrasonic welding, vibration welding, hot gas welding, and/or the like. Particularly, the welding device may be adapted to weld different materials with each other.

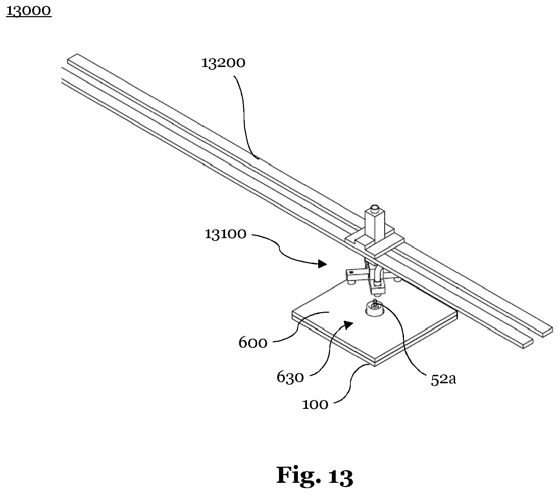

Fig. 13 schematically shows a handling manipulator 13000, at least partially. The handling manipulator 13000 is arranged movably with respect to the at least one smart tank so as to access and to control at least one smart via the access plate element 100. The handling manipulator 13000 and in particular a handling manipulator head 13100 is configured to couple with the access plate of the smart tank to control and/or operate the smart tank.

The handling manipulator 13000 may be arranged movably in all directions. Preferably, the at least one manipulator is provided on a guide rail(s) 13200 that allows movement of the manipulator 13000 in a horizontal and/or vertical plane. Here, only one guide rail 13200 is shown, however further guide rails may be provided. The manipulator may further be arranged movable in a direction perpendicular to a plane defined by the guide rails 13200. For example, the at least one manipulator may be a portal robot that comprises a first guide rail 13200 that allows moving the manipulator in a x-direction and a second guide rail (not shown) that allows moving the manipulator in a y-direction, wherein the x-direction is perpendicular to the y-direction. Additionally, or alternatively, the manipulator may comprise a third guide rail (also not shown) that allows moving the manipulator in a z-direction, wherein the z-direction is perpendicular to the x-direction and the y-direction.

The handling manipulator 13000 (respectively the manipulator head 13100) maybe moved towards a smart tank for operating the same. The handling manipulator (respectively manipulator head) may couple to a coupling portion 52a of an actuating rod 52 for opening/closing an associated valve. Thereby, the smart tank and/or the smart tank system can be operated and controlled.

Fig. 14A schematically shows a handling manipulator 14000 and in particular a manipulator head 14100 in a cut view, operating a smart tank 1. Fig. 14B shows the manipulator head 14100 in cut view, cut in a different plane. The smart tank 1 may comprise a channel 20 that ends at a port 30, which may be covered by a filter. Further, a quick connector 650 may be provided. The smart tank 1 is associated with an adaptor plate element 600 that defines an access point (here at port 30). Further, the smart tank may comprise at least one valve 50 that is associated with channel 20. The valve 50 may open channel 20 to be in communication with channel 22. The valve maybe coupled to an actuating rod 52, so as to be controllable by the handling manipulator 14000.

All channels of a smart tank, at least one channel of the smart tank and/or the reservoir(s) of the smart tank may be designed to be self-emptying. Thus, e.g. product and or cleaning fluid, such as sodium hydroxide solution can be entirely removed from the smart tank.

The handling manipulator 14000 and in particular the manipulator head 14100 of the handling manipulator 14000 is adapted to couple with the access plate element (not shown) and/or the adaptor plate element 600. The manipulator head 14100 comprises a cover element 14300 adapted to cover the adaptor plate 600 at least partially, so that at least one access point (here port 30) of the adaptor plate 600 is covered. Covering the access point reduces the risk of contamination. The cover element 14300 may comprise a sealing lip 14320 that allows to sealingly couple the cover element 14300 with the adaptor plate element 600.

Further, a UV-radiation means 14350 may be provided, preferably within the cover element 14300. The UV radiation means 14350 is adapted to irradiate at least one access point (here port 30) of the adaptor plate 600 and/or the access plate element. Using UV radiation, potential contaminants can be destroyed.

Still further a pump head or a venturi nozzle 14360 can be provided. The venturi nozzle 14360 allows to provide a negative pressure in an area of a covered access point of the adaptor plate. Thus, the covered space covered by the cover element 14300 can be evacuated and/or potential contaminants may be removed.

At least one fluid and/or gas line 14400 may be provided, for supplying and/or removing fluid or gas from the smart tank via an access point of the adaptor plate. The fluid may be an operating fluid and/or a bio-chemical fluid. In particular, the fluid may be a buffer solution and/or an acid or a base for adjusting the pH value within the smart tank. The gas may be an operating gas, such as pressurized air. Thus, the smart tank can be pressurized for controlling the flow of bio-chemical medium stored in the tank. Further, O2, N2, and/or CO2 may be supplied via a gas line. The gases may be supplied vie a common gas line or vie separate gas lines. Still further, a sensor (not shown) for measuring operating conditions of the smart tank 1, such as a pressure sensor, may be provided. This allows e.g. for integrity or leakage testing.

Further, a syringe 14200 may be provided. The syringe may be operated to remove medium from a smart tank (e.g. for sampling) and/or for providing medium to a smart tank. Further, the syringe 14200 may comprise a venting valve 14250 that may be provided with an air filter. The syringe 14200 may be coupled removably with the handling manipulator. Thus, after being used (e.g. filled), the syringe may be stored in a sampling storage means, such as a refrigerator and/or the syringe may be transferred to an analyzing means for analyzing the sample.

Fig. 15 schematically shows a handling manipulator 15000 and in particular a manipulator head 15100 in a cut view, operating a smart tank 1. A valve actuating means 15152, for actuating an actuating rod 52 of a valve of the smart tank 1 via an access point of the adaptor plate 600 is provided. The valve actuating means 15152 may be part of a drive mechanism and may be adapted to couple to an actuating means 52 of a valve of the smart tank 1, via a coupling portion 52a. Thus, the associated valve (not shown) can be controlled (e.g. opened/closed), using the handling manipulator 15000. Further, a driving means (not shown) for driving an operating means of the smart tank via an access point of the adaptor plate may also be provided. The operating means may be a stirring means. Thus, e.g. the stirring of the smart tank can be controlled, using the handling manipulator 15000.

Still further, at least one fluid and/or gas line 15400 may be provided, for supplying and/or removing fluid or gas from the smart tank via an access point of the adaptor plate.

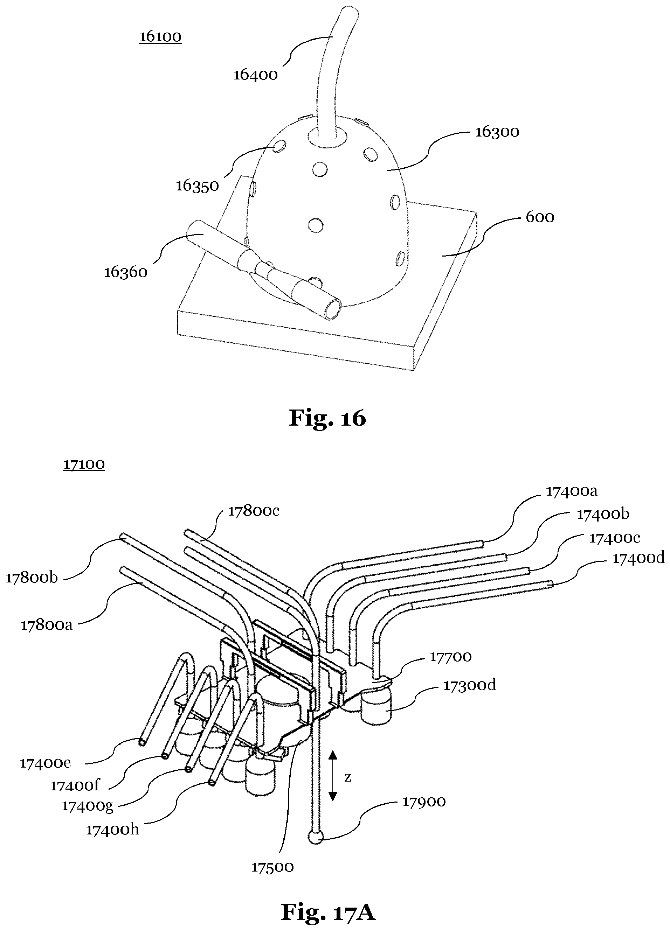

Fig. 16 schematically shows a perspective view of a further manipulator head 16100. The manipulator head 16100 comprises a cover element 16300 adapted to cover an adaptor plate 600 at least partially, so that at least one access point (not shown) of the adaptor plate 600 is covered. The manipulator head 16100 comprises multiple UV-radiation means 16350. The UV radiation means 16350 are adapted to irradiate at least one access point of the adaptor plate 600 and/or an access plate element.

Still a venturi nozzle 16360 is provided. The venturi nozzle 16360 allows to provide a negative pressure in an area of a covered access point of the adaptor plate. Thus, the covered space covered by the cover element 16300 can be evacuated and/or potential contaminants may be removed. Further, a fluid and/or gas line 16400 is provided, for supplying and/or removing fluid or gas from the smart tank via an access point of the adaptor plate.

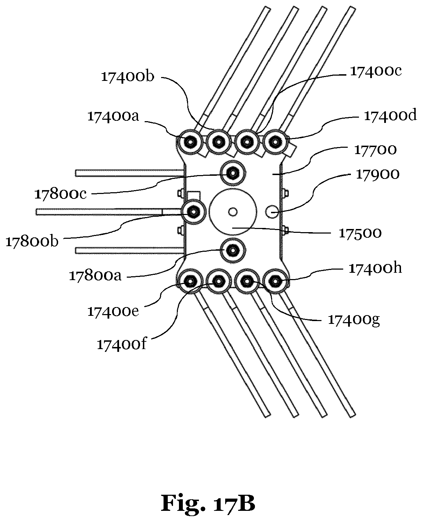

Fig. 17A schematically shows a perspective view of a manipulator head 17100. Fig. 17B shows a bottom view of the manipulator head 17100. The manipulator head 17100 comprises multiple fluid and/or gas line 17400a to 17400h, wherein each of the fluid and/or gas lines may be associated with a separate cover element 17300d. Further the manipulator head 17100 may comprise multiple valve actuating means 17800a, 17800b, 17800c, for actuating a valve 50 of the smart tank 1. Further, a driving means 17500 for driving an operating means, such as a stirring means of the smart tank is provided. Still further, at least one sensor (not shown) for measuring operating conditions of the smart tank 1, such as a pressure sensor, may be provided. Further, a spectrography means 17900 is provided. The spectrography means may be movable in z-direction to be inserted into the smart tank.

The handling manipulator may comprise at least one interchangeable manipulator head. A first interchangeable manipulator head may provide different functionality than a second interchangeable manipulator head. Different functionality may be provided by providing the respective manipulator head with different combinations of the above-mentioned means that may be part of a manipulator head. Particularly, a manipulator head, such as manipulator head 17100, may comprise a base element 17700 that is adapted to hold and support at least one or multiple of a cover element, a UV-radiation means, a pump head, a syringe, a fluid line, a gas line, a valve actuating means, a driving means, a sensor and/or a spectrography means.

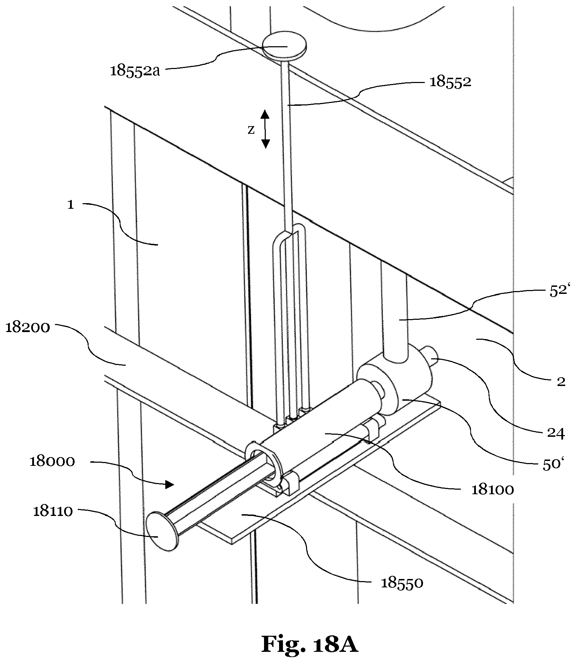

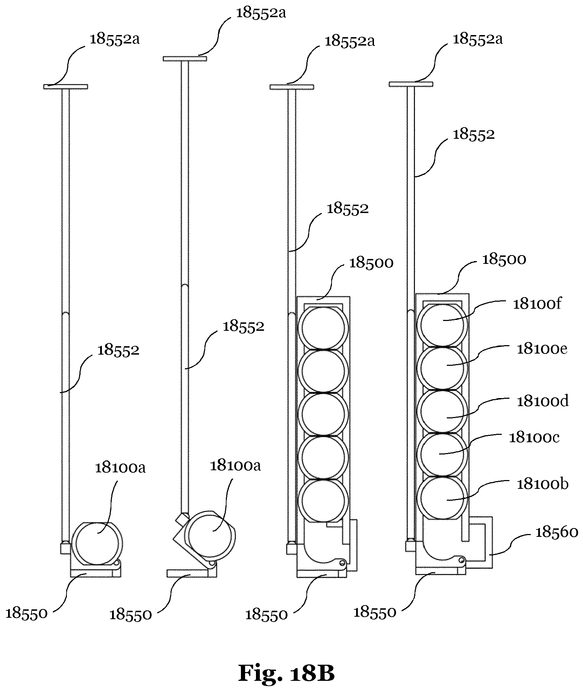

Fig. 18A schematically shows a sampling means 18000 and Fig. 18B schematically shows an operating principle of a sampling means 18000 and a sampling means magazine 18500. The system and/or the handling manipulator (particularly the manipulator head) may comprise a sampling means 18000. The sampling means is adapted to be coupled to a sampling channel 24 of the smart tank 2 for sampling from the smart tank 2. The sampling means 18000 maybe cylinder, a cartridge and/or a syringe 18100. The sample may be transferred to the sampling means 18000 by applying a positive pressure to the smart tank 2 and/or by applying a negative pressure within the sampling means 18000. In case of a syringe, negative pressure can be applied by retracting a plunger 18110. This may be done manually or automatically, e.g. by the handling manipulator. Prior to sampling an optional valve 50' that is associated with the sampling channel 24 may be opened, preferably by the handling manipulator. e.g. by operating actuating rod 52'.

The sampling means maybe adapted to be guided along multiple interconnected smart tanks 1, 2. For example, a guide rail 18200 maybe provided that guides the sampling means 18000 and/or a magazine of sampling means 18500 (cf. Fig. 18B). The guide rail 18200 may be integrally formed in sidewall elements of the smart tanks. Further, the sampling means may be guided by the handling manipulator. The sampling means may be adapted to be coupled to a sampling channel of any one of the interconnected smart tanks for sampling from the respective smart tank. Thus, samples can be taken from different smart tanks.

Particularly, the handling manipulator may be adapted to move and/or actuate the sampling means. Optionally, the sampling means 18000 may be arranged to be outputted e.g. to an air lock of a clean room bag, after being filled. The sampling means 18000 may be held in a support structure 18550, which may be part of a sampling magazine 18500. The support structure maybe coupled, preferably hinged, to an actuating means, such as an actuating rod 18552. The actuating rod can be actuated in z-direction, e.g. by a handling manipulator. Further, the actuating means 18552 may comprise a coupling portion 18552a that is preferably magnetic. Thus, a handling manipulator can couple to the actuating means, e.g. by an electro-magnet, and actuate the actuating means for outputting the sampling means as shown in Fig. 18B.

Fig. 18B shows a sampling means 18000a being held by a support structure 18550. The support structure maybe coupled, preferably hinged, to an actuating means, such as an actuating rod 18552. The actuating rod can be actuated in z-direction, e.g. by a handling manipulator. When being actuated (pulled) in z-direction, the support structure is tilted, and sampling means 18000a is outputted. The sampling magazine 18550 comprises multiple further sampling means 18000b to 18000f. By retracting locking element 18560, a further sampling means 18000b is released and can be held and supported by support structure 18550 for further sampling.

The sampling means allows to take a sample under clean room/sterile environment and to transfer the sample/filled sampling means to the outside of a clean room bag.

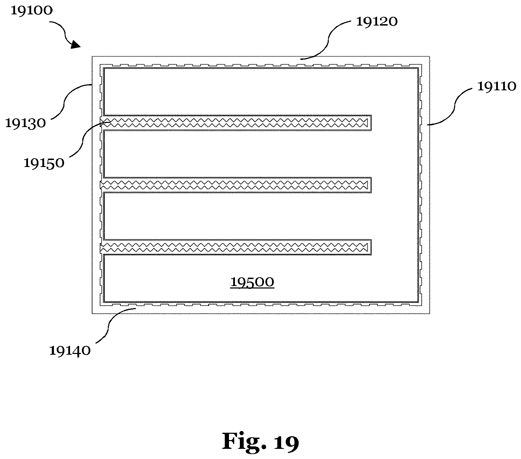

Fig. 19 shows a clean room bag 19500 in an initial state, being stored in a frame assembly 19100. The frame assembly 19100 comprise air-permeable wall elements 19110, 19120, 19130, 19140, 19150. The clean room bag 19500 comprises an air inlet port (not shown) that serves for pressurizing the stored clean room bag 19500 for leakage testing. Thus, the clean room bag 19500 can be pressurized while being stored in the frame assembly 19100 and the change of pressure over time can be measured. As the frame assembly comprises air-permeable wall elements 19110, 19120, 19130, 19140, 19150, air that leaves the stored clean room bag 19500 can also leave the frame assembly 19100 and thus, leakages can be detected. In case the change of pressure exceeds a predefined threshold, the clean room bag 19500 can be discarded as it is not as air-tight as required. The air-permeable wall elements 19110, 19120, 19130, 19140, 19150 maybe provided in form of wall elements with through holes, such as punctured sheets and/or in form of wall elements that are covered with an air-permeable means, such as an open-pored foam, a fabric, a non-woven, and/or the like.

The clean room bag 19500 may comprise an air inlet port (not shown) that serves for pressurizing the stored clean room bag 19500 for leakage testing. The air inlet port may be covered with at filter, such as a sterile filter, that allows pressurizing the assembly bag in a sterile fashion. Thus, the clean room bag 19500 can be pressurized while being stored in the frame assembly 19100 and the change of pressure over time can be measured. Particularly, leakages can be detected by comparing the measured change of pressure over time with a predefined threshold. In case the change of pressure over time exceeds the threshold, there is leakage and the clean room bag 19500 cannot be used. As the frame assembly comprises air-permeable wall elements 9110, 9120, 9130, 9140, 9150, air that leaves the stored assembly bag 9200 can also leave the frame assembly 9100 and thus, leakages can be detected.

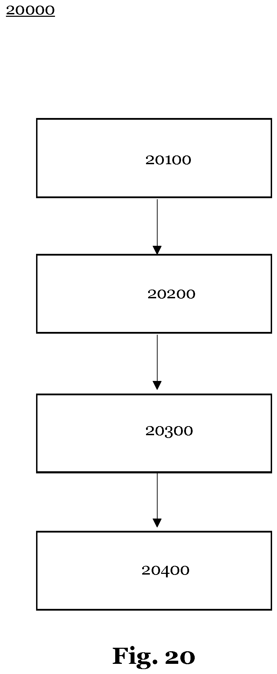

Fig. 20 schematically shows a flow diagram of a method 20000 for automatically operating at least one smart tank in a bio-pharma process line. The method 20000 comprises the step of providing 20100 a system for automatically operating at least one smart tank in a bio-pharma process line, as described above. Further, the method 20000 comprises receiving 20200 sensor signals from at least one sensor of the at least one smart tank, controlling 20300 at least one handling manipulator based on the received sensor signals and operating 20400 the at least one smart tank according to a predetermined control scheme, using the handling manipulator.

### Further embodiments:

Embodiment 1. A system for automatically operating a smart tank 1, 2 in a bio-pharma process line, the system comprising:
   at least one smart tank 1, 2, wherein the smart tank comprises an access plate element 100 that forms a wall portion of a reservoir 500 of the smart tank 1, 2 that is adapted to receive at least one biochemical medium, wherein the access plate element is configured to provide access to the smart tank, and
   at least one handling manipulator 13000, wherein the handling manipulator is arranged movably with respect to the at least one smart tank so as to access and to control at least one smart via the access plate element.
Embodiment 2. The system of embodiment 1, wherein the at least one smart tank 1, 2 is a modular smart tank that is assembled from a top plate element 100, at least one sidewall element 200, and a bottom plate element 300, wherein
   the top plate element, the at least one sidewall element and the bottom plate element are arranged to form a reservoir of the smart tank, and wherein at least one of the top plate element, the at least one sidewall element and the bottom plate element is an access plate element, wherein
   the smart tank optionally further comprises
   at least one channel 20, for guiding the at least one biochemical medium and/or an operating medium, wherein the at least one channel extends within at least one of the top plate element, the at least one sidewall element and/or the bottom plate element.
Embodiment 3. The system of any one of the preceding embodiments, wherein the at least one smart tank comprises at least one of the following components:
   a port 30, a filter 40, a valve 50, a sensor 1000 and/or an operating means, and wherein at least one of these components is accessible with the handling manipulator via the access plate element.
Embodiment 4. The system of any one of the preceding embodiments, the system further comprising
   a clean room bag 8000, 9000 that comprises at least one foil portion 8500, wherein the clean room bag is configured to receive at least one smart tank 9011, 9013 and to provide a clean room environment for the at least one smart tank when the at least one smart tank is received in the clean room bag, wherein the at least one smart tank is accessible with the handling manipulator via the access plate element, from the outside of the clean room bag.
Embodiment 5. The system of embodiment 4, the system further comprising
   at least one adaptor plate element 600, wherein the adaptor plate element is associated with at least one smart tank and can be installed on the at least one smart tank, so that one of the at least one foil portions 8500 of the clean room bag is sandwiched between the access plate element 100 of the smart tank and the adaptor plate element 600, wherein
   the adaptor plate element 600 is configured to define at least one access point 630 to provide access to the smart tank from the outside of the clean room bag, via the access plate element.
Embodiment 6. The system of any one of embodiments 4 or 5, wherein the adaptor plate element 600 is configured to cover a filter 40 and/or a port 50 of the smart tank at least partially, and wherein the foil portion 8500 is provided removably and/or pierceably in an area of the filter and/or port of the smart tank to provide access to the smart tank.
Embodiment 7. The system of any one of embodiments 4 to 6, wherein the adaptor plate element 600 is configured to support an actuating means 52 of a valve 50 and/or an operating means of the smart tank, and wherein the foil portion is provided removably and/or pierceably in an area of the actuating means of a valve and/or an operating means of the smart tank to provide access to the smart tank.
Embodiment 8. The system of the preceding embodiment, wherein the handling manipulator comprises a manipulator head 13100, 14100, 16100, 17100, wherein the manipulator head is adapted to couple with the access plate element and/or the adaptor plate element, and wherein the manipulator head comprises at least one of the following:
   a cover element 14300, 16300, 17300d adapted to cover the adaptor plate at least partially, so that at least one access point of the adaptor plate is covered;
   a UV-radiation means 14350, 16350, the UV radiation means being adapted to irradiate at least one access point of the adaptor plate and/or the access plate element;
   a pump head 14360, 16360, for providing a negative pressure in an area of a covered access point of the adaptor plate,
   a fluid line 17400a to 17400h, for supplying and/or removing fluid from the smart tank via an access point of the adaptor plate,
   a gas line 17400a to 17400h, for supplying and/or removing gas from the smart tank, via an access point of the adaptor plate,
   a valve actuating means 17800a to 17800c, for actuating a valve of the smart tank via an access point of the adaptor plate,
   a driving means 17500 for driving an operating means of the smart tank via an access point of the adaptor plate,
   a sensor for measuring operating conditions of the smart tank, such as a pressure sensor, and/or
   a spectrography means 17900.
Embodiment 9. The system of the preceding embodiment, wherein the handling manipulator 13000 comprises at least one interchangeable manipulator head 13100, 14100, 16100, 17100, and wherein a first interchangeable manipulator head may provide different functionality than a second interchangeable manipulator head.
Embodiment 10. The system of any one of the preceding embodiments, wherein the handling manipulator 13000 is adapted to weld the foil portion 8500 with the adaptor plate 600 and/or the access plate 100.
Embodiment 11. The system of any one of the preceding embodiments, wherein the handling manipulator 13000 is adapted to weld and/or seal a hose portion of the smart tank.
Embodiment 12. The system of any one of the preceding embodiments, further comprising a sampling means 18000, the sampling means is adapted to be coupled to a sampling channel 24 of the smart tank 2 for sampling from the smart tank, wherein optionally the sample is transferred to the sampling means by a positive pressure applied in the smart tank , the sampling means may comprise at least one syringe.
Embodiment 13. The system of the preceding embodiment, wherein the sampling means 18000 is adapted to be guided along multiple interconnected smart tanks, and wherein the sampling means is adapted to be coupled to a sampling channel of any one of the interconnected smart tanks for sampling from the respective smart tank.
Embodiment 14. The system of the any one of embodiments 12 or 13, wherein the handling manipulator 13000 is adapted to move and/or actuate the sampling means 18000.
Embodiment 15. The system of any one of embodiments 12 to 14, wherein the system further comprises a sampling means magazine 18500, for receiving multiple sampling means, and wherein the sampling means magazine is adapted to output a filled sampling means after sampling, wherein the sampling means magazine optionally outputs the filled sampling means to an air lock of the clean room bag 8000, 9000 and/or a cooled storing device.
Embodiment 16. The system of the preceding embodiment, wherein the smart tanks have different height dimensions, the system further comprising at least one height compensation means 9012, 9014, that is adapted to be coupled to at least one of the multiple smart tanks 9011, 9013, so that the access plate elements of the multiple smart tanks can be installed at substantially the same height, wherein the height compensation means optionally is provided within a bottom plate element of the smart tank.
Embodiment 17. The system of any one of the preceding embodiments, further comprising a control unit, the control unit is adapted to receive sensor signals from at least one sensors 1000 of the at least one smart tank and to control the at least one handling manipulator based on the received sensor signals to control the at least smart tank according to a predetermined control scheme.
Embodiment 18. A clean room bag 8000, 9000, for being used in system according to any one of the previous system embodiments,
   the clean room bag 8000, 9000 being configured to receive at least one smart tank 9011, 9013 and to provide a clean room environment for the at least one smart tank when the at least one smart tank is received in the clean room bag, wherein the clean room bag comprises at least one foil portion 8550, that is adapted to be sandwiched between an access plate element 100 of the smart tank and an adaptor plate element 800, so as to define at least one access point to provide access to the smart tank from the outside of the clean room bag, via the access plate element.
Embodiment 19. The clean room bag 8000, 9000 of embodiment 18, wherein the clean room bag is adapted to sealingly couple to an assembly room that servers for assembling at least one smart tank, wherein the clean room bag is further configured to receive the at least one assembled smart tank from the assembly room.
Embodiment 20. The clean room bag 8000, 9000 of any one of embodiments 18 or 19, wherein the clean room bag is a sterile bag, that is optionally a single-use bag.
Embodiment 21. The clean room bag 8000, 9000 of any one of embodiments 18 to 20, wherein the clean room bag is held by a support structure 8100, 9100, and/or wherein the clean room bag is inflatable.
Embodiment 22. The clean room bag 8000, 9000 of any one of embodiments 18 to 21, wherein the clean room bag 8000, 9000 comprises a multi-ply outer shell, wherein a first ply of the shell is the outermost ply, and wherein a second ply of the shell is the innermost ply, and wherein the space surrounded by the second ply of the shell is pressurized with a pressure being lower than a pressure provided between the first and the second ply of the shell, wherein a colored gas may be provided between the first and the second ply of the shell.
Embodiment 23. The clean room bag 8000, 9000 of any one of embodiments 18 to 22, wherein the clean room bag includes an sealable opening 8200 that is configured for receiving assembled smart tanks, wherein the sealable opening of the clean room bag is configured to open jointly with a corresponding sealable opening of an assembly room, when the clean room bag is sealingly coupled to the assembly room.
Embodiment 24. The clean room bag 8000, 9000 of embodiment 23, wherein the sealable opening 8200 of the clean room bag is covered by at least one cover element 8210, and wherein the at least one cover element is adapted to couple with a corresponding cover element of a corresponding sealable opening of an assembly room that is covered by the at least one corresponding cover element, to open jointly with the corresponding cover element.
Embodiment 25. The clean room bag 8000, 9000 of any one of embodiments 23 or 24, further comprising a sealing frame 8300, 9310, 9320, 9330, the sealing frame being configured to frame the sealable opening of the clean room bag, wherein the sealing frame may further comprise a UV-light source, the UV-light source is associated with the sealable opening of the clean room bag.
Embodiment 26. The clean room bag 8000, 9000 of any one of embodiments 18 to 25, wherein the clean room bag comprises at least one rail 9015, 9025, 9035, 9045 for guiding at least one received assembled smart tank within the clean room bag.
Embodiment 27. The clean room bag 8000, 9000 of any one of embodiments 18 to 26, wherein the clean room bag 8000, 9000 further comprises at least one height compensation means, that is adapted to be coupled to a received smart tank, and to adjust the height of the smart tank, so that an access plate element of the smart tank is installed at substantially the same height of a further smart tank received within the clean room bag .
Embodiment 28. The clean room bag 19500 of any one of embodiments 18 to 27, wherein the clean room bag is initially stored in a frame assembly 19100, the frame assembly comprising air-permeable wall elements 19110, 19120, 19130, 19140, 19150, and wherein the clean room bag comprises an air inlet port that serves for pressurizing the stored clean room bag for leakage testing .
Embodiment 30. A handling manipulator 13000 for automatically operating a smart tank 1, 2 in a bio-pharma process line in a system according to any one of embodiments 1 to 17,
   the handling manipulator is adapted to be movably with respect to at least one smart tank so as to access and to control at least one smart via an access plate element.
Embodiment 31. The handling manipulator 13000 of embodiment 30, further comprising a manipulator head, wherein the manipulator head is adapted to couple with the access plate element and/or the adaptor plate element, and wherein the manipulator head comprises at least one of the following:
   a cover element 14300, 16300, 17300d adapted to cover the adaptor plate at least partially, so that at least one access point of the adaptor plate is covered;
   a UV-radiation means 14350, 16350, the UV radiation means being adapted to irradiate at least one access point of the adaptor plate and/or the access plate element;
   a pump head 14360, 16360, for providing a negative pressure in an area of a covered access point of the adaptor plate,
   a fluid line 17400a to 17400h, for supplying and/or removing fluid from the smart tank via an access point of the adaptor plate,
   a gas line 17400a to 17400h, for supplying and/or removing gas from the smart tank, via an access point of the adaptor plate,
   a valve actuating means 17800a to 17800c, for actuating a valve of the smart tank via an access point of the adaptor plate,
   a driving means 17500 for driving an operating means of the smart tank via an access point of the adaptor plate,
   a sensor for measuring operating conditions of the smart tank, such as a pressure sensor, and/or
   a spectrography means 17900, wherein
   the manipulator head may be an interchangeable manipulator head.
Embodiment 32. The handling manipulator 13000 of any one of embodiments 39 to 31, wherein the handling manipulator is adapted to weld the foil portion with the adaptor plate and/or the access plate and/or wherein the handling manipulator is adapted to weld and/or seal a hose portion of the smart tank.
Embodiment 33. A method 20000 for automatically operating at least one smart tank in a bio-pharma process line, the method comprising:
   providing 20100 a system according to any one of embodiment 1 to 17,
   receiving 20200 sensor signals from at least one sensor of the at least one smart tank;
   controlling 20300 at least one handling manipulator based on the received sensor signals
   operating 20400 the at least one smart tank according to a predetermined control scheme, using the handling manipulator.

## Claims

1. A system for automatically operating a smart tank (1, 2) in a bio-pharma process line, the system comprising:
at least one smart tank (1, 2), wherein the smart tank comprises an access plate element (100) that forms a wall portion of a reservoir (500) of the smart tank (1, 2) that is adapted to receive at least one biochemical medium, wherein the access plate element is configured to provide access to the smart tank, and
at least one handling manipulator (13000), wherein the handling manipulator is arranged movably with respect to the at least one smart tank so as to access and to control at least one smart via the access plate element.

2. The system of claim 1, wherein the at least one smart tank (1, 2) is a modular smart tank that is assembled from a top plate element (100), at least one sidewall element (200), and a bottom plate element (300), wherein
the top plate element, the at least one sidewall element and the bottom plate element are arranged to form a reservoir of the smart tank, and wherein at least one of the top plate element, the at least one sidewall element and the bottom plate element is an access plate element, wherein
the smart tank optionally further comprises
at least one channel (20), for guiding the at least one biochemical medium and/or an operating medium, wherein the at least one channel extends within at least one of the top plate element, the at least one sidewall element and/or the bottom plate element.

3. The system of any one of the preceding claims, wherein the at least one smart tank comprises at least one of the following components:
a port (30), a filter (40), a valve (50), a sensor (1000) and/or an operating means, and wherein at least one of these components is accessible with the handling manipulator via the access plate element.

4. The system of any one of the preceding claims, the system further comprising
a clean room bag (8000, 9000) that comprises at least one foil portion (8500), wherein the clean room bag is configured to receive at least one smart tank (9011, 9013) and to provide a clean room environment for the at least one smart tank when the at least one smart tank is received in the clean room bag, wherein the at least one smart tank is accessible with the handling manipulator via the access plate element, from the outside of the clean room bag,
the system optionally further comprising
at least one adaptor plate element (600), wherein the adaptor plate element is associated with at least one smart tank and can be installed on the at least one smart tank, so that one of the at least one foil portions (8500) of the clean room bag is sandwiched between the access plate element (100) of the smart tank and the adaptor plate element (600), wherein
the adaptor plate element (600) is configured to define at least one access point (630) to provide access to the smart tank from the outside of the clean room bag, via the access plate element, wherein further optionally
the adaptor plate element (600) is configured to cover a filter (40) and/or a port (50) of the smart tank at least partially, and wherein the foil portion (8500) is provided removably and/or pierceably in an area of the filter and/or port of the smart tank to provide access to the smart tank and/or, wherein the adaptor plate element (600) is configured to support an actuating means (52) of a valve (50) and/or an operating means of the smart tank, and wherein the foil portion is provided removably and/or pierceably in an area of the actuating means of a valve and/or an operating means of the smart tank to provide access to the smart tank.

5. The system of the preceding claim, wherein the handling manipulator comprises a manipulator head (13100, 14100, 16100, 17100), wherein the manipulator head is adapted to couple with the access plate element and/or the adaptor plate element, and wherein the manipulator head comprises at least one of the following:
a cover element (14300, 16300, 17300d) adapted to cover the adaptor plate at least partially, so that at least one access point of the adaptor plate is covered;
a UV-radiation means (14350, 16350), the UV radiation means being adapted to irradiate at least one access point of the adaptor plate and/or the access plate element;
a pump head (14360, 16360), for providing a negative pressure in an area of a covered access point of the adaptor plate,
a fluid line (17400a to 17400h), for supplying and/or removing fluid from the smart tank via an access point of the adaptor plate,
a gas line (17400a to 17400h), for supplying and/or removing gas from the smart tank, via an access point of the adaptor plate,
a valve actuating means (17800a to 17800c), for actuating a valve of the smart tank via an access point of the adaptor plate,
a driving means (17500) for driving an operating means of the smart tank via an access point of the adaptor plate,
a sensor for measuring operating conditions of the smart tank, such as a pressure sensor, and/or
a spectrography means (17900).

6. The system of any one of the preceding claims, wherein the handling manipulator (13000) is adapted to weld the foil portion (8500) with the adaptor plate (600) and/or the access plate (100).

7. The system of any one of the preceding claims, further comprising a sampling means (18000), the sampling means is adapted to be coupled to a sampling channel (24) of the smart tank (2) for sampling from the smart tank, wherein optionally the sample is transferred to the sampling means by a positive pressure applied in the smart tank, the sampling means may comprise at least one syringe, wherein optionally
the handling manipulator (13000) is adapted to move and/or actuate the sampling means (18000), wherein even further optionally
the system further comprises a sampling means magazine (18500), for receiving multiple sampling means, and wherein the sampling means magazine is adapted to output a filled sampling means after sampling, wherein
the sampling means magazine optionally outputs the filled sampling means to an air lock of the clean room bag (8000, 9000) and/or a cooled storing device.

8. A clean room bag (8000, 9000), for being used in system according to any one of the previous system claims,
the clean room bag (8000, 9000) being configured to receive at least one smart tank (9011, 9013) and to provide a clean room environment for the at least one smart tank when the at least one smart tank is received in the clean room bag, wherein
the clean room bag comprises at least one foil portion (8550), that is adapted to be sandwiched between an access plate element (100) of the smart tank and an adaptor plate element (800), so as to define at least one access point to provide access to the smart tank from the outside of the clean room bag, via the access plate element.

9. The clean room bag (8000, 9000) of claim 8, wherein the clean room bag is adapted to sealingly couple to an assembly room that servers for assembling at least one smart tank, wherein the clean room bag is further configured to receive the at least one assembled smart tank from the assembly room, and/or wherein the clean room bag is a sterile bag, that is optionally a single-use bag.

10. The clean room bag (8000, 9000) of any one of claims 8 or 9, wherein the clean room bag (8000, 9000) comprises a multi-ply outer shell, wherein a first ply of the shell is the outermost ply, and wherein a second ply of the shell is the innermost ply, and wherein the space surrounded by the second ply of the shell is pressurized with a pressure being lower than a pressure provided between the first and the second ply of the shell, wherein a colored gas may be provided between the first and the second ply of the shell.

11. The clean room bag (8000, 9000) of any one of claims 8 to 10, wherein the clean room bag includes an sealable opening (8200) that is configured for receiving assembled smart tanks, wherein the sealable opening of the clean room bag is configured to open jointly with a corresponding sealable opening of an assembly room, when the clean room bag is sealingly coupled to the assembly room., wherein optionally
the sealable opening (8200) of the clean room bag is covered by at least one cover element (8210), and wherein the at least one cover element is adapted to couple with a corresponding cover element of a corresponding sealable opening of an assembly room that is covered by the at least one corresponding cover element, to open jointly with the corresponding cover element, and wherein further optionally,
the clean room bag (8000, 9000) comprises a sealing frame (8300, 9310, 9320, 9330), the sealing frame being configured to frame the sealable opening of the clean room bag, wherein the sealing frame may further comprise a UV-light source, the UV-light source is associated with the sealable opening of the clean room bag.

12. The clean room bag (8000, 9000) of any one of claims 8 to 11, wherein the clean room bag comprises at least one rail (9015, 9025, 9035, 9045) for guiding at least one received assembled smart tank within the clean room bag.

13. The clean room bag (19500) of any one of claims 8 to 12, wherein the clean room bag is initially stored in a frame assembly (19100), the frame assembly comprising air-permeable wall elements (19110, 19120, 19130, 19140, 19150), and wherein the clean room bag comprises an air inlet port that serves for pressurizing the stored clean room bag for leakage testing.

14. A handling manipulator (13000) for automatically operating a smart tank (1, 2) in a bio-pharma process line in a system according to any one of claims 1 to 7,
the handling manipulator is adapted to be movably with respect to at least one smart tank so as to access and to control at least one smart via an access plate element, wherein the handling manipulator (13000) may optionally comprise a manipulator head, wherein the manipulator head is adapted to couple with the access plate element and/or the adaptor plate element, and wherein the manipulator head comprises at least one of the following:
a cover element (14300, 16300, 17300d) adapted to cover the adaptor plate at least partially, so that at least one access point of the adaptor plate is covered;
a UV-radiation means (14350, 16350), the UV radiation means being adapted to irradiate at least one access point of the adaptor plate and/or the access plate element;
a pump head (14360, 16360), for providing a negative pressure in an area of a covered access point of the adaptor plate,
a fluid line (17400a to 17400h), for supplying and/or removing fluid from the smart tank via an access point of the adaptor plate,
a gas line (17400a to 17400h), for supplying and/or removing gas from the smart tank, via an access point of the adaptor plate,
a valve actuating means (17800a to 17800c), for actuating a valve of the smart tank via an access point of the adaptor plate,
a driving means (17500) for driving an operating means of the smart tank via an access point of the adaptor plate,
a sensor for measuring operating conditions of the smart tank, such as a pressure sensor, and/or
a spectrography means (17900), wherein
the manipulator head may be an interchangeable manipulator head.

15. A method (20000) for automatically operating at least one smart tank in a bio-pharma process line, the method comprising:
providing (20100) a system according to any one of claim 1 to 7,
receiving (20200) sensor signals from at least one sensor of the at least one smart tank;
controlling (20300) at least one handling manipulator based on the received sensor signals
operating (20400) the at least one smart tank according to a predetermined control scheme, using the handling manipulator.
